# EUROPEAN PATENT SPECIFICATION

(11) **EP 1 389 236 B1**
(45) Date of publication and mention of the grant of the patent: **18.10.2006**
(21) Application number: 02712231.6
(22) Date of filing: 20.02.2002
(51) Int. Cl.: C12N 15/13, C12N 15/63, C12N 15/11, C12N 5/10, C07K 14/705, C07K 16/28, G01N 33/68, A61K 35/28, A61K 31/7088, A61P 17/02, A61P 35/00, C07K 16/00

(54) **IMMUNOGLOBULIN SUPERFAMILY VARIANTS EXPRESSED IN MESENCHYMAL CELLS AND THERAPEUTIC USES THEREOF**
VARIANTEN DER IMMUNGLOBULIN SUPERFAMILIE EXPRIMIERT IN MESENCHYMALEN ZELLEN UND DEREN THERAPEUTISCHE VERWENDUNGEN
ALLELES DE LA SUPERFAMILLE DES IMMUNOGLOBULINES EXPRIMES DANS DES CELLULES MESENCHYMATEUSES ET LEURS UTILISATIONS THERAPEUTIQUES

(30) Priority: 20.02.2001 IL 14153901; 25.09.2001 IL 14565801
(43) Date of publication of application: 18.02.2004
(73) Proprietor: YEDA RESEARCH AND DEVELOPMENT CO. LTD., Rehovot 76100 (IL)
(72) Inventor: ZIPORI, Dov, 76574 Rehovot (IL); SHAV-TAL, Yaron, 44814 Elkanah (IL); BARDA-SAAD, Mira, 55900 Ganei Tiqvah (IL)
(74) Representative: Becker Kurig Straus
(86) International application number: PCT/IL2002/000129
(87) International publication number: WO 2002/066648

(56) References cited:
- WO-A-99/45031
- US-A- 5 591 669
- WOLFGANG CURT D ET AL: "TARP: A nuclear protein expressed in prostate and breast cancer cells derived from an alternate reading frame of the T cell receptor gamma chain locus." PROCEEDINGS OF THE NATIONAL ACADEMY OF SCIENCES OF THE UNITED STATES, vol. 97, no. 17, 15 August 2000 (2000-08-15), pages 9437-9442, XP002207096 August 15, 2000 ISSN: 0027-8424 cited in the application
- BARDA-SAAD MIRA ET AL: "The mesenchyme expresses T cell receptor mRNAs: relevance to cell growth control." ONCOGENE. ENGLAND 27 MAR 2002, vol. 21, no. 13, 27 March 2002 (2002-03-27), pages 2029-2036, XP001088472 ISSN: 0950-9232
- ALESSANDRINI A ET AL: "COORDINATION OF IMMUNOGLOBULIN DJH TRANSCRIPTION AND D-TO-JH REARRANGEMENT BY PROMOTER-ENHANCER APPROXIMATION" MOLECULAR AND CELLULAR BIOLOGY, AMERICAN SOCIETY FOR MICROBIOLOGY, WASHINGTON, US, vol. 11, no. 4, April 1991 (1991-04), pages 2096-2107, XP002930987 ISSN: 0270-7306
- SCHREIER P H ET AL: "Allotypic differences in murine mu genes." NUCLEIC ACIDS RESEARCH. ENGLAND 11 MAR 1986, vol. 14, no. 5, 11 March 1986 (1986-03-11), pages 2381-2389, XP001088614 ISSN: 0305-1048
- MARKS R ET AL: "TRUNCATED MU MU' CHAINS IN MURINE IMMUNOGLOBULIN M EVIDENCE THAT MU' CHAINS LACK VARIABLE REGIONS" JOURNAL OF EXPERIMENTAL MEDICINE, vol. 162, no. 6, 1985, pages 1862-1877, XP001088625 ISSN: 0022-1007

### SEQUENCE LISTING

<110> YEDA RESEARCH AND DEVELOPMENT Co. Ltd. at the Weizmann Institute of Science
<120> IMMUNOGLOBULIN SUPERFAMILY VARIANTS EXPRESSED IN MESENCHYMAL CELLS AND THERAPEUTIC USES THEREOF
<130> YEDA/010 PCT
<140> PCT/IL02/00129
   <141> 2002-02-20
<150> 141539
   <151> 2001-02-20
<150> 145658
   <151> 2001-09-25
<160> 42
<170> PatentIn version 3.1
<210> 1
   <211> 89
   <212> DNA
   <213> Mus musculus
<400> 1
   atgggttttt gtacacccac taaaggggtc tatgatagtg tgactacttt gactactggg 60
   gccaaggcac cactctcaca gtctcctca 89
<210> 2
   <211> 30
   <212> PRT
   <213> Mus musculus
<400> 2
   Met Gly Phe Cys Thr Pro Thr Lys Gly Val Tyr Asp Ser Val Thr Thr
   1 5 10 15
   Leu Thr Thr Gly Ala Lys Ala Pro Leu Ser Gln Ser Pro Ser
   20 25 30
<210> 3
   <211> 1479
   <212> DNA
   <213> Mus musculus
<220>
   <221> misc_feature
   <222> (1)..(42)
   <223> joining region 4 (J4)
<220>
   <221> misc_feature
   <222> (43)..(1350)
   <223> constant domain
<220>
   <221> misc_feature
   <222> (1351)..(1479)
   <223> transmembrane domain
<400> 3
   atggactact ggggtcaagg aacctcagtc accgtctcct cagagagtca gtccttccca      60
   aatgtcttcc ccctcgtctc ctgcgagagc cccctgtctg ataagaatct ggtggccatg     120
   ggctgcctgg cccgggactt cctgcccagc accatttcct tcacctggaa ctaccagaac     180
   aacactgaag tcatccaggg tatcagaacc ttcccaacac tgaggacagg gggcaagtac     240
   ctagccacct cgcaggtctt gctgtctccc aagagcatcc ttgaaggttc agatgaatac     300
   cttgtatgca aaatccacta cggaggcaaa aacagagatc tgcatgtgcc cattccagct     360
   gtcgcagaga tgaatcccaa tgtaaatgtg ttcgtcccac cacgggatgg cttctctggc     420
   cctgcaccac gcaagtctaa actcatctgc gaggccacga acttcactcc aaaaccgatc     480
   acagtatcct ggctaaagga tgggaagctc gtggaatctg gcttcaccac agatccggtg     540
   accatcgaga acaaaggatc cacaccccaa acctacaagg tcataagcac acttaccatc     600
   tctgaaatcg actggctgaa cctgaatgtg tacacctgcc gtgtggatca caggggtctc     660
   accttcttga agaacgtgtc ctccacatgt gctgccagag gtccctccac agacatccta     720
   accttcacca tccccccctc ctttgccgac atcttcctca gcaagtccgc taacctgacc     780
   tgtctggtct caaacctggc aacctatgaa accctgaata tctcctgggc ttctcaaagt     840
   ggtgaaccac tggaaaccaa aattaaaatc atggaaagcc atcccaatgg caccttcagt     900
   gctaagggtg tggctagtgt ttgtgtggaa gactggaata acaggaagga atttgtgtgt     960
   actgtgactc acagggatct gccttcgcca cagaagaaat tcatctcaaa acccaatgag    1020
   gtgcacaaac atccacctgc tgtgtacctg ctgccaccag ctcgtgagca actgaaccta    1080
   agagagtcag ccacagtcac ctgcctggtg aagggcttct ctcctgcaga catcagtgtg    1140
   cagtggcttc agagagggca actcttgccc caagagaagt atgtgaccag tgccccgatg    1200
   ccagagcctg gggccccagg cttctacttt acccacagca tcctgactgt gacagaggag    1260
   gaatggaact ccggagagac ctatacctgc gttgtagggc acgaggccct gccacaccta    1320
   gtgaccgaga ggaccgtgga caagtccact gagggggagg tgaatgctga ggaggaaggc    1380
   tttgagaacc tgtggaccac tgcctccacc ttcaccttca tcgtcctctt cctcctgagc    1440
   ctcttctaca gcaccgtcac cctgttcaag gtgaaatga                           1479
<210> 4
   <211> 1413
   <212> DNA
   <213> Mus musculus
<220>
   <221> misc_feature
   <222> (1)..(42)
   <223> joining region 4 (J4)
<220>
   <221> misc_feature
   <222> (43)..(1350)
   <223> constant region
<220>
   <221> misc_feature
   <222> (1351)..(1413)
   <223> cytoplasmic domain
<400> 4
   atggactact ggggtcaagg aacctcagtc accgtctcct cagagagtca gtccttccca     60
   aatgtcttcc ccctcgtctc ctgcgagagc cccctgtctg ataagaatct ggtggccatg    120
   ggctgcctgg cccgggactt cctgcccagc accatttcct tcacctggaa ctaccagaac    180
   aacactgaag tcatccaggg tatcagaacc ttcccaacac tgaggacagg gggcaagtac    240
   ctagccacct cgcaggtctt gctgtctccc aagagcatcc ttgaaggttc agatgaatac    300
   cttgtatgca aaatccacta cggaggcaaa aacagagatc tgcatgtgcc cattccagct    360
   gtcgcagaga tgaatcccaa tgtaaatgtg ttcgtcccac cacgggatgg cttctctggc    420
   cctgcaccac gcaagtctaa actcatctgc gaggccacga acttcactcc aaaaccgatc    480
   acagtatcct ggctaaagga tgggaagctc gtggaatctg gcttcaccac agatccggtg    540
   accatcgaga acaaaggatc cacaccccaa acctacaagg tcataagcac acttaccatc    600
   tctgaaatcg actggctgaa cctgaatgtg tacacctgcc gtgtggatca caggggtctc    660
   accttcttga agaacgtgtc ctccacatgt gctgccagag gtccctccac agacatccta    720
   accttcacca tccccccctc ctttgccgac atcttcctca gcaagtccgc taacctgacc    780
   tgtctggtct caaacctggc aacctatgaa accctgaata tctcctgggc ttctcaaagt    840
   ggtgaaccac tggaaaccaa aattaaaatc atggaaagcc atcccaatgg caccttcagt    900
   gctaagggtg tggctagtgt ttgtgtggaa gactggaata acaggaagga atttgtgtgt    960
   actgtgactc acagggatct gccttcgcca cagaagaaat tcatctcaaa acccaatgag   1020
   gtgcacaaac atccacctgc tgtgtacctg ctgccaccag ctcgtgagca actgaaccta   1080
   agagagtcag ccacagtcac ctgcctggtg aagggcttct ctcctgcaga catcagtgtg   1140
   cagtggcttc agagagggca actcttgccc caagagaagt atgtgaccag tgccccgatg   1200
   ccagagcctg ggccccaggg cttctacttt acccacagca tcctgactgt gacagaggag   1260
   gaatggaact ccggagagac ctatacctgc gttgtagggc acgaggccct gccacaccta   1320
   gtgaccgaga ggaccgtgga caagtccact ggtaaaccca cactgtacaa tgtctccctg   1380
   atcatgtctg acacaggcgg cacctgctat tga                                1413
<210> 5
   <211> 1362
   <212> DNA
   <213> Mus musculus
<220>
   <221> misc_feature
   <222> (1)..(1233)
   <223> constant domain
<220>
   <221> misc_feature
   <222> (1234)..(1362)
   <223> transmembrane domain
<400> 5 <210> 6
   <211> 1296
   <212> DNA
   <213> Mus musculus
<220>
   <221> misc_feature
   <222> (1)..(1233)
   <223> constant region
<220>
   <221> misc_feature
   <222> (1234)..(1296)
   <223> cytoplasmic domain
<400> 6 <210> 7
   <211> 26
   <212> PRT
   <213> Mus musculus
<400> 7 <210> 8
   <211> 19
   <212> PRT
   <213> Mus musculus
<400> 8
<210> 9
   <211> 4
   <212> PRT
   <213> Mus musculus
<400> 9 <210> 10
   <211> 19
   <212> PRT
   <213> Mus musculus
<400> 10
<210> 11
   <211> 6
   <212> PRT
   <213> Mus musculus
<400> 11
   Met Glu Cys Leu Thr Val
   1 5
<210> 12
   <211> 3
   <212> PRT
   <213> Mus musculus
<400> 12
   Met Thr Val
   1
<210> 13
   <211> 13
   <212> PRT
   <213> Mus musculus
<400> 13
   Met Cys Gly Ser Glu Glu Val Phe Val Val Glu Ser Ala
   1 5 10
<210> 14
   <211> 92
   <212> PRT
   <213> Mus musculus
<400> 14
   Met Ala Cys Tyr Gln Met Tyr Phe Thr Gly Arg Lys Val Asp Glu Pro
   1 5 10 15
   Ser Glu Leu Gly Ser Gly Leu Glu Leu Ser Tyr Phe His Thr Gly Gly
   20 25 30
   Ser Ser Gln Ala Val Gly Leu Phe Ile Glu Asn Met Ile Ser Thr Ser
   35 40 45
   His Gly His Phe Gln Glu Met Gln Phe Ser Ile Trp Ser Phe Thr Val
   50 55 60
   Leu Gln Ile Ser Ala Pro Gly Ser His Leu Val Pro Glu Thr Glu Arg
   65 70 75 80
   Ala Glu Gly Pro Gly Val Phe Val Glu His Asp Ile
   85 90
<210> 15
   <211> 87
   <212> PRT
   <213> Mus musculus
<400> 15
   Met Tyr Phe Thr Gly Arg Lys Val Asp Glu Pro Ser Glu Leu Gly Ser
   1               5                   10                  15
   Gly Leu Glu Leu Ser Tyr Phe His Thr Gly Gly Ser Ser Gln Ala Val
               20                  25                  30
   Gly Leu Phe Ile Glu Asn Met Ile Ser Thr Ser His Gly His Phe Gln
           35                  40                  45
   Glu Met Gln Phe Ser Ile Trp Ser Phe Thr Val Leu Gln Ile Ser Ala
       50                  55                  60
   Pro Gly Ser His Leu Val Pro Glu Thr Glu Arg Ala Glu Gly Pro Gly
   65                  70                  75                  80
   Val Phe Val Glu His Asp Ile
                   85
<210> 16
   <211> 49
   <212> PRT
   <213> Mus musculus
<400> 16
   Met Ile Ser Thr Ser His Gly His Phe Gln Glu Met Gln Phe Ser Ile
   1               5                   10                  15
   Trp Ser Phe Thr Val Leu Gln Ile Ser Ala Pro Gly Ser His Leu Val
               20                  25                  30
   Pro Glu Thr Glu Arg Ala Glu Gly Pro Gly Val Phe Val Glu His Asp
           35                  40                  45
   Ile
<210> 17
   <211> 38
   <212> PRT
   <213> Mus musculus
<400> 17
   Met Gln Phe Ser Ile Trp Ser Phe Thr Val Leu Gln Ile Ser Ala Pro
   1               5                   10                  15
   Gly Ser His Leu Val Pro Glu Thr Glu Arg Ala Glu Gly Pro Gly Val
               20                  25                  30
   Phe Val Glu His Asp Ile
           35
<210> 18
   <211> 21
   <212> PRT
   <213> Mus musculus
<400> 18
   Met Trp Trp Gly Leu Ile Leu Ser Ala Ser Val Lys Phe Leu Gln Arg
   1               5                   10                  15
   Lys Glu Ile Leu Cys
               20
<210> 19
   <211> 14
   <212> PRT
   <213> Mus musculus
<400> 19
   Met Val Gly Ala Asp Leu Cys Lys Gly Gly Trp His Cys Val
   1               5                   10
<210> 20
   <211> 13
   <212> PRT
   <213> Mus musculus
<400> 20
   Met Arg Glu Pro Val Lys Asn Leu Gln Gly Leu Val Ser
   1               5                   10
<210> 21
   <211> 25
   <212> PRT
   <213> Mus musculus
<400> 21
   Met Glu Val Tyr Glu Leu Arg Val Thr Leu Met Glu Thr Gly Arg Glu
   1               5                   10                  15
   Arg Ser His Phe Val Lys Thr Ser Leu
               20                  25
<210> 22
   <211> 15
   <212> PRT
   <213> Mus musculus
<400> 22
   Met Glu Thr Gly Arg Glu Arg Ser His Phe Val Lys Thr Ser Leu
   1 5 10 15
<210> 23
   <211> 30
   <212> PRT
   <213> Homo sapiens
<400> 23
   Met Gly Leu Ser Ala Val Gly Arg Thr Arg Ala Glu Ser Gly Thr Ala
   1 5 10 15
   Glu Arg Ala Ala Pro Val Phe Val Leu Gly Leu Gln Ala Val
   20 25 30
<210> 24
   <211> 24
   <212> PRT
   <213> Homo sapiens
<400> 24
   Met Leu Leu Trp Asp Pro Ser Gly Phe Gln Gln Ile Ser Ile Lys Lys
   1 5 10 15
   Val Ile Ser Lys Thr Leu Pro Thr
   20
   <210> 25
   <211> 26
   <212> PRT
   <213> Homo sapiens
<400> 25
   Met Leu Pro Asn Thr Met Gly Gln Leu Val Glu Gly Gly His Met Lys
   1 5 10 15
   Gln Val Leu Ser Lys Ala Val Leu Thr Val
   20 25
<210> 26
   <211> 21
   <212> PRT
   <213> Homo sapiens
<400> 26
   Met Gly Gln Leu Val Glu Gly Gly His Met Lys Gln Val Leu Ser Lys
   1               5                   10                  15
   Ala Val Leu Thr Val
               20
<210> 27
   <211> 12
   <212> PRT
   <213> Homo sapiens
<400> 27
   Met Lys Gln Val Leu Ser Lys Ala Val Leu Thr Val
   1               5                   10
<210> 28
   <211> 4
   <212> PRT
   <213> Homo sapiens
<400> 28
   Met Ser Glu Cys
   1
<210> 29
   <211> 11
   <212> PRT
   <213> Homo sapiens
<400> 29
   Met Ala His Phe Val Ala Val Gln Ile Thr Val
   1               5                   10
<210> 30
   <211> 6
   <212> PRT
   <213> Homo sapiens
<400> 30
   Met Gly Ile Cys Tyr Ser
   1               5
<210> 31
   <211> 18
   <212> PRT
   <213> Homo sapiens
<400> 31
   Met Lys Arg Ala Gly Glu Gly Lys Ser Phe Cys Lys Gly Arg His Tyr
   1 5 10 15
   Ser Val
<210> 32
   <211> 21
   <212> PRT
   <213> Homo sapiens
<400> 32
   Met Leu Thr Thr Leu Ile Tyr Tyr Gln Gly Asn Ser Val Ile Phe Val
   1 5 10 15
   Arg Gln His Ser Ala
   20
<210> 33
   <211> 37
   <212> PRT
   <213> Homo sapiens
<400> 33
   Met Gln Leu Pro His Phe Val Ala Arg Leu Phe Pro His Glu Gln Phe
   1 5 10 15
   Val Phe Ile Gln Gln Leu Ser Ser Leu Gly Lys Pro Phe Cys Arg Gly
   20 25 30
   Val Cys His Ser Val
   35
<210> 34
   <211> 11
   <212> PRT
   <213> Homo sapiens
<400> 34
   Met Gly Phe Ser Lys Gly Arg Lys Cys Cys Gly
   1 5 10
<210> 35
   <211> 18
   <212> PRT
   <213> Homo sapiens
<400> 35
   Met Lys Lys Ile Trp Leu Ser Arg Lys Val Phe Leu Tyr Trp Ala Glu
   1 5 10 15
   Thr Leu
<210> 36
   <211> 34
   <212> PRT
   <213> Homo sapiens
<400> 36
   Met Gly Lys Val His Val Met Pro Leu Leu Phe Met Glu Ser Lys Ala
   1 5 10 15
   Ala Ser Ile Asn Gly Asn Ile Met Leu Val Tyr Val Glu Thr His Asn
   20 25 30
   Thr Val
<210> 37
   <211> 28
   <212> PRT
   <213> Homo sapiens
<400> 37
   Met Pro Leu Leu Phe Met Glu Ser Lys Ala Ala Ser Ile Asn Gly Asn
   1 5 10 15
   Ile Met Leu Val Tyr Val Glu Thr His Asn Thr Val
   20 25
<210> 38
   <211> 23
   <212> PRT
   <213> Homo sapiens
<400> 38
   Met Glu Ser Lys Ala Ala Ser Ile Asn Gly Asn Ile Met Leu Val Tyr
   1 5 10 15
   Val Glu Thr His Asn Thr Val
   20
<210> 39
   <211> 11
   <212> PRT
   <213> Homo sapiens
<400> 39
   Met Leu Val Tyr Val Glu Thr His Asn Thr Val
   1 5 10
<210> 40
   <211> 55
   <212> PRT
   <213> Homo sapiens
<400> 40
   Met Glu Glu Gly Ser Phe Ile Tyr Thr Ile Lys Gly Pro Trp Met Thr
   1 5 10 15
   His Ser Leu Cys Asp Cys Cys Val Ile Gly Phe Gln Thr Leu Ala Leu
   20 25 30
   Ile Gly Ile Ile Gly Glu Gly Thr Trp Trp Leu Leu Gln Gly Val Phe
   35 40 45
   Cys Leu Gly Arg Thr His Cys
   50 55
<210> 41
   <211> 41
   <212> PRT
   <213> Homo sapiens
<400> 41
   Met Thr His Ser Leu Cys Asp Cys Cys Val Ile Gly Phe Gln Thr Leu
   1 5 10 15
   Ala Leu Ile Gly Ile Ile Gly Glu Gly Thr Trp Trp Leu Leu Gln Gly
   20 25 30
   Val Phe Cys Leu Gly Arg Thr His Cys
   35 40
<210> 42
   <211> 16
   <212> PRT
   <213> Homo sapiens
<400> 42
   Met Glu Ser Gln Ala Thr Gly Phe Cys Tyr Glu Ala Ser His Ser Val
   1 5 10 15

## Description

### FIELD OF THE INVENTION

The present invention relates to novel transcripts of immunoglobulin superfamily genes, particularly T cell receptor (TCR) and immunoglobulin heavy chain variants expressed on mesenchymal and endothelial cells, said variants useful in mediating intercellular interactions between the mesenchyme and/or endothelium and other cell types, thereby regulating stem cell growth and differentiation.

### BACKGROUND OF THE INVENTION

### T cell receptors

Major Histocompatibility Complex (MHC)-restricted T cells express heterodimeric surface protein receptors (αβTCR) co-localized with up to five additional non-variant membrane receptors (Strominger, 1989; Abbas et al., 1994; Jameson et al., 1995). This TCR complex specifically binds processed peptide antigens associated with MHC molecules. The interactions of TCR with MHC bound peptides on various target cells may have consequences both in terms of T cell proliferation and in activation of effector mechanisms leading to target cell killing, graft rejection, and other biological effects.

Functional TCR α and β chain genes, which are capable of being expressed as polypeptides, are normally present only in cells of the T lymphocyte lineage. These functional TCR genes are formed by somatic rearrangement of germline gene segments. Each TCR locus consists of variable (V), joining (J), and constant (C) region genes, and the β chain locus contains diversity (D) gene segments. In mice there are 20 to 30 Vβ gene segments that are located 5' of the two clusters of C and J segments. There is a single Cα gene associated with a large 5' cluster of up to 50 different J segments and about 75 Vα segments. There is a large region of intervening DNA between Vα and Jα exons, which includes the entire TCR δ chain locus. During maturation of T cells in the thymus, the TCR segments are rearranged in a defined order, resulting in the formation of functional TCRα and β genes in which V, D, J and C segments are in close proximity to each other.

The β chain locus rearranges prior to the α locus. The primary transcripts contain noncoding intronic sequences between the VDJ and C genes, which are later spliced out. The functional T cell receptor is comprised of 2 polypeptides: the α chain is a 40 to 60 kD acidic glycoprotein, and the β chain is a 40 to 50 kD uncharged or basic glycoprotein. The V and C regions of α and β chains form intrachain disulfide bond loops, which might contribute to the formation of a tertiary structure and are presented on the cell membrane. The C region contains the transmembrane domain and a short cytoplasmic tail thought to be too small to have intrinsic signal transducing properties.

T cells (Qian et al., 1993; Yoshikai et al., 1984) as well as B cells (Calman and Peterlin, 1986) express a series of incomplete transcripts of TCRα and β, that vary in size and structure. These transcripts may be out of frame or their sequence may contain many stop codons. In some cases mRNAs encoding the constant region flanked by an upstream spliced J segment were identified. In one case such a transcript of human TCRβ, which contains an in-frame codon for methionine, has been reported (Fagioli et al., 1991). However, no evidence for the existence of a protein encoded by these transcripts in T cells has been documented. TCR transcripts have also been reported in cell lineages other than T or B lymphocytes. Thus, TCRα mRNA was identified in murine kidney (Madrenas et al., 1991; Madrenas et al., 1992; Madrenas et al., 1994). A recent study identified in epithelial tumor cells a partial TCRγ chain mRNA, lacking the V region. This mRNA encodes a 7 kDa protein, TARP, which is translated from an alternate reading frame and is therefore not homologous to the TCRγ protein (Essand et al., 1999; Wolfgang et al., 2000). No evidence for TCRαβ or TCRδ transcripts or proteins was found in that study. It is therefore generally accepted that TCRβ transcripts are not found outside of the lymphocyte lineage and that TCR protein expressed at the cell surface is a specific T cell trait.

Nevertheless, it is possible that the TCR gene family serves functions other than those already ascribed to it. The laboratories of the present inventors and colleagues have now discovered that primary mesenchyme, as well as mesenchymal cell clones, express T cell receptor (TCR)αβ mRNAs, lacking the variable region. Immunological and genetic evidence support the expression of a corresponding TCRβ protein. This truncated TCR protein is implicated in the regulation of mesenchymal cell growth (Barda-Saad et al. 2002).

### The pre B cell receptor (preBCR)

In the bone marrow, B cell development can be divided into different stages, based on the rearrangement status of the IgH and IgL chain loci (Ehlich et al 1994; ten Boekel et al 1997) and the expression of intracellular and surface-bound markers. The pre-B cell receptor consists of immunoglobulin µ heavy chains and surrogate light chain, the VpreB and λ5 proteins (Hardy et al 1991).

Immunoglobulins (Igs) are synthesized exclusively by B lymphocytes (Abbas et al 1994). The immunoglobulin molecule can exist in two very different environments: at the cell membrane as a surface antigen receptor and in solution as a secreted antibody. The immunoglobulin molecule is composed of two identical light chains and two identical heavy chains. The light and heavy chains can each be divided into an N terminal variable (V) and a C terminal constant (C) region. The V regions are responsible for antigen binding, whereas the C regions embody the various effector functions of the molecule. The various classes of immunoglobulins with different functions (IgM, IgD, IgG, IgA, IgE) are distinguished by different heavy chains (µ, δ, γ, α, ε), with the difference residing in their C_{H} regions (Cµ, Cδ, Cγ Cα, Cε) (Rogers et al 1980).

B lymphocytes mature from hemopoietic stem cells through a series of developmental stages that are characterized by sequential DNA rearrangements of Ig gene segments. The rearrangement of Ig genes allows B cells to respond to a wide spectrum of foreign antigens (Ags). The V, D and J segments encoding parts of the IgH and the V and J segments of IgL-chains are rearranged in a stepwise fashion (Melchers, & Rolink 1999). ProB cells begin to rearrange D_{H} to J_{H} segments of the H chain locus, so that in PreBI cells (B220⁺, c-kit ⁺) both H-chain alleles are D_{H}J_{H} rearranged. ProB and PreBI cells already produce surrogate light chains VpreB and λ5 in preparation for the formation of the preBCR (Melchers et al 1993). When V_{H} to D_{H} to J_{H} rearrangements are initiated in PreBI cells, those rearrangements that are in frame will generate a functional IgH chain gene.

The formation of the preBCR has a functional consequence for precursor B cells. PreBII cells are stimulated to undergo between two and five rounds of divisions (Rolink et al 2000) and to expand the number of µH chain producing preBII cells in which, subsequently, L-chain rearrangements are initiated. The preBCR signals for the inhibition of rearrangements at the second D_{H} J_{H} - rearranged H chain allele (allelic exclusion) (Ehlich et al 1994; ten Boekel et al 1997). Subsequent processing of the RNA leads to splicing out of the intron between the VDJ complex and the most proximal C region gene, which is the C- giving rise to a functional mRNA for the µ heavy chain.

The recombination activating genes, RAG-1 and RAG-2, are essential for V(D)J recombination (Shinkai et al 1992, Mombaerts et al 1992). During B lineage development in adult mice, RAG-1 and RAG-2 are expressed exclusively in early B progenitors of the bone marrow and expression ceases prior to the migration of B lineage cells from the bone marrow (Hardy et al 1991; Osmond 1990). Furthermore, mice that lack either RAG-1 or RAG-2 fail to develop mature lymphocytes due to their inability to initiate rearrangement of the antigen receptor genes (Shinkai et al 1992; Mombaerts et al 1992). However, expression of a rearranged µHC transgene in the RAG-deficient background partially rescued this developmental block in the B lineage, leading to the generation of B220⁺CD43⁻ pre-B cells, demonstrating that µ chain expression was sufficient to drive this developmental transition (Young et al 1994, Spanopoulou et al 1994).

µ chains of membrane (µₘ) and secreted (µₛ) forms differ in structure. The µₘ chain is larger than the µₛ chain and has hydrophobic properties not exhibited by the µₛ (Rogers et al 1980). An essential role for components of the preBCR complex has been established. Targeted disruption of the membrane exons of the µH chain, or the λ5 locus, result in the failure of normal B cell development and the loss of allelic exclusion in pre-B cells (Kitamura et al 1991; Kitamura et al 1992a; Kitamura et al 1992b; Loffert et al 1996). PreB cells can express µs chains as well as, µₘ chains providing a potential source for a soluble form of preBCR. The µₛ chains can associate with SLC and assemble into a soluble preBCR complex in preB cells. µₛ chains can associate with SLC internally, but are efficiently retained and degraded. Mutation of a single cysteine (Cys575) in the µₛ tailpiece (tp) results in the release of soluble preBCR from the endoplasmic reticulum (ER) and its subsequent secretion.

The soluble preBCR does not bind the hapten recognized by antibody (Ab) consisting of the same heavy chain V region paired with a conventional L chain, consistent with the preBCR having a unique specificity (Bornemann et al 1997).

Because the preBCR, like the mature BCR, has no known intrinsic enzymatic functions, it must rely upon associated proteins to provide a functional linkage with intracellular signaling pathways. The mature and preBCR- associated Igα and Igβ chains contain immunoreceptor tyrosine-based activation motifs (ITAMs), which are targets for phosphorylation by tyrosine kinases (Reth 1984); these proteins are required for normal B cell development (Gong & Nussenzweig 1996; Torres et al 1996). Furthermore, the importance of an ITAM-associated tyrosine kinase activity during early B lymphopoiesis was demonstrated in mice deficient in the syk tyrosine kinase, in which an incomplete block in development was observed at the B220⁺CD43⁺ proB cell stage (Cheng, et al 1995; Turner et al 1995).

### Truncated heavy chain Dµ

Reth & Alt discovered in 1984 (Reth and Alt 1984) a truncated Dµ heavy chain in a permanent lymphoid cell line, which represents a pre B stage of B-lymphocytes, by transformation of bone marrow or fetal calf liver cells with Abelson murine leukemia virus (A-MuLV). Some A-MuLV generated lines produce an unusually small µ heavy chain mRNA and sometimes a small µ protein. The short µ mRNA sequences arise from the transcription of DJ_{H} rearrangements and the short µ proteins from the translation of the resulting DJ_{H} Cµ containing mRNAs (Dµ mRNA). Due to an inexact joining mechanism, the D_{H} can be rearranged to the J_{H} in three possible reading frames (RFs). A majority of the D_{H} segments carry their own promoter and an ATG translational initiation codon. When the D_{H} is rearranged to a J_{H} in RF2, according to the nomenclature of. (Ichihara et al 1989), this D_{H}J_{H} complex can be translated into a truncated µ chain protein. The size of these small µ chains was analyzed by Western blot using anti-IgM antisera and ¹²⁵I-labelled monoclonal IgM antibody. Lysates from control transformant express normal-sized µ-chains of 70Kd molecular weight while cell lines express an abnormally small µ protein of approximately 57Kd. Furthermore, instead of normal 2.4 and 2.7 kb µ mRNAs which encode, respectively, the secreted and membrane- bound forms of the µ proteins, cell lines 300-19 and 298-13 (Reth and Alt 1984) contain truncated Cµ-specific RNAs of 2.0 and 2.3 kb; these species contain 3' ends specific to the membrane and secreted forms of the protein, respectively. D_{µ} preBCR can mediate a block in B cell development, probably by inhibiting V_{H} to D_{H}J_{H} rearrangements, as well as inducing V_{L} to J_{L} rearrangements (Tornberg et al 1998, Home et al 1996).

There is an unmet need for and it would be advantageous to have polypeptide or peptide markers for mesenchymal cells that are involved in control of proliferation and differentiation of hemopoietic stem cells. In addition, it would be advantageous to develop interventive therapeutic strategies based either on gene therapy or antisense molecular therapy to treat disorders involving the proliferation and differentiation of hemopoietic stem cells.

Citation of any document herein is not intended as an admission that such document is pertinent prior art, or considered material to the patentability of any claim of the present application. Any statement as to content or a date of any document is based on the information available to the applicant at the time of filing and does not constitute an admission as to the correctness of such a statement.

### SUMMARY OF THE INVENTION

It is an object of the present invention to provide polypeptide or peptide markers for mesenchymal cells that are involved in control of proliferation and differentiation of hemopoietic stem cells. It is another object of the present invention to provide methods for intervening therapeutically utilizing methods of gene therapy or antisense molecular therapy to treat disorders involving the proliferation and differentiation of hemopoietic stem cells.

The present invention discloses novel transcripts of Immunoglobulin (Ig) superfamily genes, particularly T cell receptor (TCR) and Ig heavy chain variants, expressed on mesenchymal and endothelial cells which are mediators of intercellular interactions leading, either directly or indirectly, to modulation in the proliferation and differentiation of their neighboring cells.

More preferably, the mesenchymal TCR or Ig variants are either directly or indirectly involved in the regulation of stem cell growth and differentiation. The therapeutic uses of these molecules are also disclosed.

The growth and differentiation of normal cells and malignant tumors within different tissues, are all dependent on mesenchymal cellular interactions, as is known in the art. It is now disclosed that the lack of the TCR in mesenchymal cells causes loss of the ability of the mesenchyme to support hemopoiesis.

The present invention also relates to truncated immunoglobulin (Ig) transcripts and encoded proteins found in mesenchymal and endothelial stromal cells as detailed herein below, and to the uses of these molecules.

The present invention relates, in one aspect, to cDNA molecules encoded by immunoglobulin genes, said cDNA molecules lacking V region and comprising a constant (C) domain and a joining (J) region sequences, and a 5' intronic J sequence upstream said J region sequence including an in-frame methionine codon. The novel polynucleotides of the invention are exemplified herein by transcripts of Ig µ chains, expressed in mesenchymal and endothelial cells.

The novel polynucleotide sequences disclosed herein and the corresponding proteins, polypeptides or peptides encoded by these polynucleotide sequences may be derived from any mammalian species including human genetic material.

In one embodiment of the invention, the cDNA molecule encodes a truncated µ heavy chain transcript from the mouse cell line MBA-2.1 with the sequence: which codes for the peptide:
MGFCTPTKGVYDSVTTLTTGAKAPLSQSPS [SEQ ID NO:2]

In another embodiment of the invention, the truncated µ heavy chain 5'end begins from a joining region 4 (J4), and comprises a constant region and a transmembrane domain:
atg gac tac tgg ggt caa gga acc tca gtc acc gtc tcc tca

### Constant

### Transmembrane domain

In another embodiment of the invention, the truncated µ heavy chain 5'end comprises a joining region 4 (J4), a constant region and a cytoplasmic domain:
atg gac tac tgg ggt caa gga acc tca gtc acc gtc tcc tca

### Constant

### Cytoplasmic domain

In another embodiment of the invention, the truncated µ heavy chain 5'end extends from the constant region and comprises the transmembrane domain:

### Transmembrane domain

In another embodiment of the invention, the truncated µ heavy chain 5'end begins from the constant region and comprises the cytoplasmic domain:

### Cytoplasmic domain

In another aspect, the invention relates to antisense DNA molecules of the cDNA molecules of the invention described hereinabove.

The invention further relates to expression vectors comprising the cDNA and antisense molecules of the invention, and to host cells, particularly mammalian cells, comprising said vectors. In one preferred embodiment the host cells are transfected mesenchymal or endothelial human cells.

The cDNA of the invention can be used to transfect mesenchymal and endothelial human cells for mediating their intercellular functions. Thus, the invention relates to compositions comprising said transfected mesenchymal and endothelial human cells for use in disorders requiring modulation of their intercellular functions, such as wound healing

The invention further relates to a method for modulating mesenchymal and endothelial intercellular functions comprising the step of administering to a subject in need thereof transfected mesenchymal and endothelial human cells comprising a cDNA molecule according to the invention, in an amount effective to augment their intercellular communication. Preferably, these transfected mesenchymal or endothelial cells are autologous cells.

According to one currently preferred embodiment these methods are applicable for inducing or enhancing wound healing. According to another currently preferred embodiment, this method is applicable for augmenting hemopoiesis in autologous or allogeneic bone marrow transplantation. These methods can be carried out as *in vitro, ex vivo* or *in vivo* procedures, especially in the form of gene therapy.

Alternatively and preferably, antisense molecules of the invention are useful in the treatment of malignant diseases in general where there is a tendency for the carcinoma cells to spread to the patient's bone marrow. The method can be carried out as an *in vitro, ex vivo* or *in vivo* procedure, especially in the form of gene therapy, or antisense therapy.

The antisense DNA molecules of the invention can be used to transfect mesenchymal and endothelial human cells for inhibiting or suppressing their intercellular interactions. Thus the invention relates to compositions comprising said transfected mesenchymal and endothelial human cells for use in disorders requiring inhibition or suppression of their intercellular interactions, such as in carcinomas.

The invention further relates to a method for suppressing mesenchymal and endothelial cell growth comprising the step of administering to a subject in need thereof antisense DNA, a vector comprising this DNA or transfected mesenchymal and endothelial human cells comprising an antisense DNA molecule of the invention, in an amount effective to suppress their intercellular interactions, such as for suppression of carcinomas. Preferably these transfected mesenchymal or endothelial cells will be autologous.

According to the present invention mesenchymal TCR or Ig transcripts may be either directly or indirectly involved in the regulation of stem cell growth and differentiation. It is anticipated that additional molecular variants of the Ig superfamily will be transcribed in and expressed by mesenchymal and/or endothelial cells and these too are within the scope of the present invention. It will be appreciated by the skilled artisan that additional molecules may be involved in molecular complexes that regulate intercellular interactions together with the novel truncated variants of the present invention. It is also understood that the regulatory effect of the molecules of the invention may be either direct or indirect, the latter term expressing the need for additional molecular mediators or signals to achieve the observed biological effect.

The novel polynucleotide sequences disclosed herein and the corresponding proteins, polypeptides or peptides encoded by these polynucleotide sequences may be derived from any mammalian species including human genetic material.

### BRIEF DESCRIPTION OF THE DRAWINGS

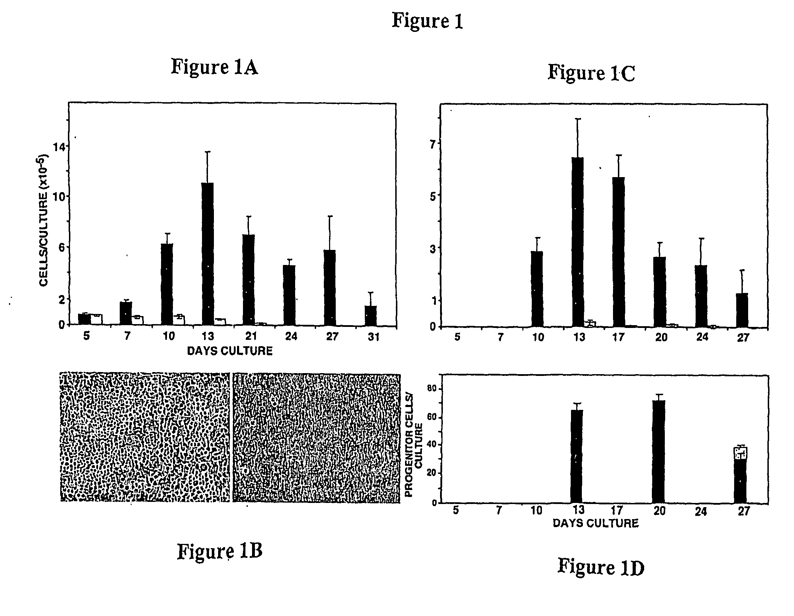

**Figure 1.** Defective hemopoietic supporting activity of MEF from TCRβ deficient mice. (A) Long term proliferation of hemopoietic cells in the liquid phase of co-cultures of bone marrow cells seeded onto confluent cultures of MEF (9^{th} *in vitro* passage) from normal embryos (full bars) and from TCRβ^{-/-} mice (empty bars). (B) A photomicrograph of similar co-cultures in which 10³ FACS sorted CD34⁺ Lin⁻ cells were seeded onto wild type control MEF (I) as compared to TCRβ^{-/-} MEF (II) (C) Proliferation of hemopoietic cells in the liquid phase of cultures seeded with CD34⁺ Lin⁻ cells. **(D)** Determinations of hemopoietic colony forming cells in the liquid phase of the cultures shown in (C) at the indicated time points, and at the end of one month culture, within the adherent cell layers (gray bar). Figures are determinations of triplicate cultures ± standard deviation of the mean.
**Figure 2.** µ, heavy chain mRNA transcript detection by Northern blot analysis. Total RNA and poly A+ RNA samples were hybridized with µ heavy chain probe. Lane 1 mouse spleen total RNA; Lane 2 MBA-2.1 total RNA; Lane 3 MBA-2.1 poly A+ RNA.
**Figure 3.** Immunoblot analysis of µ heavy chain with goat anti mouse- µ chain. Cells lysates were loaded on 10% SDS gel and then transferred to nitrocellulose membrane. The membrane was blotted with goat anti-mouse-µ chain. Lane 1- 70Z cells; Lane 2 - MBA 2.4; Lane 3 - MBA-2.1.
Figure 4. Immunostaining of MBA-2.1 cells with goat anti mouse- µ chain and amplified with biotinylated donkey anti goat and ABC kit.

### DETAILED DESCRIPTION OF THE INVENTION

The present invention discloses the transcription of novel variants of members of the genes referred to as the Immunoglobulin superfamily, in cells that do not belong to the hemopoietic or lymphoid lineages. Hitherto these molecules were considered to be specific to lymphoid lineages, with the exception of certain transformed cell lines or tumors that were known to express certain abnormal transcripts of these genes. Importantly, the novel transcripts now discovered in mesenchymal cells are also translated and expressed as novel truncated variants of TCR and Ig molecules by these cells.

These novel truncated variants are capable of regulating cell growth and differentiation, as well as mediating cell-cell interactions. These attributes can be used to stimulate cell growth, for instance especially in order to enhance hemopoiesis. These methods should prove particularly useful in situations involving bone marrow transplantation, by way of example. In contradistinction, these attributes can be used to suppress cell growth, for instance in order to prevent cancer growth or metastasis. The growth stimulation might entail gene therapy, while the growth suppression might entail either antisense therapy or antibody targeting or other methods known in the art.

The present invention resulted in part from studies on the interactions of stromal cell lines with thymic T cells, during which we used reverse transcription polymerase chain reaction (RT-PCR) to amplify TCR gene fragments. Unexpectedly, the MBA-13 mesenchymal stromal cell line, derived from mouse bone marrow, was found to consistently express TCRβ constant (Cβ) region, while cDNA from a negative control tissue, i.e. liver, and from several control cell lines such as pre-B cells, plasmacytoma and mastocytoma cells, did not produce PCR products using primers from the TCR gene.

Further studies with a variety of stromal cell lines, showed the existence of TCR gene derived mRNAs that encode short versions of the gene consisting of the constant (C) domain, which is identical to that of T cell receptor, a joining (J) region, which may be one of several alternatives, and a 5' sequence corresponding to an intronic J sequence (again one of several alternatives) including an in-frame codon for methionine (see Barda-Saad et al. 2002). This mRNA lacks V region sequences. One of such molecules, namely a new version of a TCRβ2.6, was shown to exist in mesenchymal cells and to encode a cell surface mesenchymal protein. Expression on the mRNA level has also been observed in the thymus (see Barda-Saad et al. 2002). Table 1 below provides examples of the peptides encoded by the intronic J sequences of these novel transcripts.

The finding that mesenchymal cells express TCR genes raised the possibility that other members of the immunoglobulin (Ig) superfamily are expressed in the mesenchyme. We screened a series of stromal mesenchymal cell lines derived in our laboratory including one subtype that shares properties with endothelial cells (MBA-2.1 cells). Based on our experience with truncated TCR molecules, which were found to be lacking the variable part and possessing a J region preceded by an intronic sequence including a codon for methionine, we performed a PCR analysis on MBA-2.1 cells and found that they do express mRNA transcripts corresponding to truncated Ig µ heavy chains. We thus find that at least one type, and possibly more, of stromal cells express the Ig µ chains and may present this protein as a surface molecule.

We further disclose the ability of the truncated immunoglobulin superfamily variants expressed in mesenchymal cells to regulate or modulate growth/differentiation control of their neighboring cells. In other words, not only do the novel molecules of the invention modulate the growth of the mesenchymal cells themselves but they are also capable of regulating the growth and differentiation of hemopoietic stem cells. Moreover, they are capable of regulating the growth of transformed cells.

The present invention discloses the novel uses of the truncated TCR variants as well as the uses of the novel truncated Ig variants.

**Table 1 Truncated TCR variant transcripts from mesenchymal cells comprise intronic J sequences encoding deduced peptides.**

| |
|---|
| M E N V S N P G S C I E E G E E R G R I L G S P F L [SEQ ID NO:7] |
| |
| M G E Y L A E P R G F V C G V E P L C [SEQ ID NO:8] |
| |
| M A W H [SEQ ID NO:9] |
| |
| M E A G W E V Q H W V SD M E C L T V [SEQ ID NO:10] |
| |
| M E C L T V [SEQ ID NO:11] |
| |
| M T V [SEQ ID NO:12] |
| |
| M C G S E E V F V V E S A [SEQ ID NO:13] |
| |
| M A C Y Q M Y F T G R K V D E P S E L G S G L |
| E L S Y F H T G G S S Q A V G L F I E N M I S T S |
| H G H F Q E M Q F S I W S F T V L Q I S A P G S H |
| L V P E T E R A E G P G V F V E H D I [SEQ ID NO:14] |
| |
| M Y F T G R K V D E P S E L G S G L |
| E L S Y F H T G G S S Q A V G L P I E N M I S T S |
| H G H F Q E M Q F S I W S F T V L Q I S A P G S H |
| L V P E T E R A E G P G V F V E H D I [SEQ ID NO:15] |
| |
| M I S T S H G H F Q E M Q F S I W S F T V L Q I S |
| A P G S H L V P E T E R A E G P G V F V E H D I [SEQ ID NO:16] |
| |
| M Q F S I W S F T V L Q I S A P G S H L V P E T E R A E G P G V F |
| V E H D I [SEQ ID NO:17] |
| |
| M W W G L I L S A S V K F L Q R K E I L C [SEQ ID NO:18] |
| |
| M V G A D L C K G G W H C V [SEQ ID NO:19] |
| |
| M R E P V K N L Q G L V S [SEQ ID NO:20] |
| |
| M E V Y E L R V T L M E T G R E R S H F V K T S L [SEQ ID |
| NO:21] |
| |
| M E T G R E R S H F V K T S L [SEQ ID NO:22] |
| |
| M G L S A V G R T R A E S G T A E R A A P V F V L G L Q A V |
| [SEQ ID NO:23] |
| |
| M L L W D P S G F Q Q I S I K K V I S K T L P T [SEQ ID NO:24] |
| |
| M L P N T M G Q L V E G G H M K Q V L S K A V L T V [SEQID |
| NO:25] |
| |
| M G Q L V E G G H M K Q V L S K A V L T V [SEQ ID NO:26]; |
| |
| M K Q V L S K A V L T V [SEQ ID NO:27]; |
| |
| M S E C [SEQ ID NO:28]; |
| |
| M A H F V A V Q I T V [SEQ ID NO:29]; |
| |
| M G I C Y S [SEQ ID NO:30]; |
| |
| M K R A G E G K S F C K G R H Y S V [SEQ ID NO:31]; |
| |
| M L T T L I Y Y Q G N S V I F V R Q H S A [SEQ ID NO:32]; |
| |
| M Q L P H F V A R L F P H E Q F V F I Q Q L S S L G K P F C R G V |
| C H S V [SEQ ID NO:33]; |
| |
| M G F S K G R K C C G [SEQ ID NO:34]; |
| |
| M K K I W L S R K V F L Y W A E T L [SEQ ID NO:35]; |
| |
| M G K V H V M P L L F M E S K A A S I N G N I M L V Y V E T H N |
| T V [SEQ ID NO:36]; |
| |
| M P L L F M E S K A A S I N G N I M L V Y V E T H N T V [SEQ ID |
| NO:37]; |
| |
| M E S K A A S I N G N I M L V Y V E T H N T V [SEQ ID NO:38]; |
| M L V Y V E T H N T V [SEQ ID NO:39]; |
| |
| M E E G S F I Y T I K G P W M T H S L C D C C V I G F Q T L A L I |
| G I I G E G T W W L L Q G V F C L G R T H C [SEQ ID NO:40]; |
| |
| M T H S L C D C C V I G F Q T L A L I G I I G E G T W W L L Q G V |
| F C L G R T H C [SEQ ID NO:41]; |
| |
| M E S Q A T G F C Y E A S H S V [SEQ ID NO:42]. |

Thus, both TCR and Ig chain, are now disclosed herein to be linked to the cell-cell interactions, cell growth and differentiation and thus can be used to control stromal functions. The TCR appears to be most abundant in mesenchymal stroma whereas the µ chain seems to be abundant in endothelial stroma.

It is anticipated that additional molecular variants of the Ig superfamily will be transcribed and expressed on mesenchymal and/or endothelial cells and these too are within the scope of the present invention. It will be appreciated by the skilled artisan that additional molecules may be involved in molecular complexes that regulate intercellular interactions together with the novel truncated variants of the present invention.

### The endothelium

The cellular and molecular mechanisms that allow for the maintenance of hemopoietic stem cells are inadequately understood. Morphological examination of various embryonic hemopoietic sites revealed that hemopoietic progenitor cells are in close physical contact with the endothelium in both yolk sac and aorta-gonadomesonephros region (AGM) (Lin et al 1995). The close association in the development of hemopoietic and endothelial cells during embryonic life (Garcia Porrero et al. 1995) has led to the hypothesis that the two lineages may derive from a common precursor called hemangioblast. Recently several authors reported that endothelial cells, both vascular endothelial cells and bone marrow endothelial cells, support hemopoiesis (Bagdy & Heinrich 1991). The mechanism by which the endothelial cells support hemopoiesis is thought to involve endothelial cell derived cytokines (Fleischman et al 1995), extracellular matrix proteins (Rafii et al 1994) and cell-cell interactions (Fina et al 1994). Stomal cells are thought to be an essential component of the lymphohematopoietic microenvironment. B lymphocytes develop in the liver during fetal life and in the bone marrow of adult animals (Kincade et al 1981). It has been suspected that yet unknown stromal cell molecules may be involved in B-lineage cell growth and development (Palacios and_Samaridis 1992).

### Mesenchymal cells

Mesenchymal cells play a central role in embryogenesis by directing organogenesis. In the adult organism, tissue remodeling, such as that occurring in wound healing, is initiated by mesenchymal fibroblasts. The study of regulation of hemopoiesis demonstrated that blood cell formation is locally regulated by stromal mesenchyme (Zipori, 1989; Zipori et al., 1989; Zipori, 1990; Weintroub et al., 1996). Indeed, bone marrow-derived primary stroma as well as a variety of mesenchymal cells lines derived from primary bone marrow cultures exhibit an in vitro capacity to support hemopoiesis and, upon transplantation, promote the formation of bone and hemopoietically active tissue in vivo at the site of transplantation. The molecules that mediate the instructive stromal activities have been shown to be a variety of cytokines and adhesion molecules. However, the molecules identified thus far cannot account for the wide spectrum of stromal cell functions and certainly do not explain stroma organization, stem cell renewal and other vital stromal functions.

Mesenchymal cells from the bone marrow are well known to be obligatory for the maintenance and renewal of hemopoietic stem cells *in vitro,* and these cells are critical for the maintenance of hemopoiesis *in vivo.* This function of the mesenchyme is not restricted to blood cells. In fact, every tissue and organ is composed of a stromal mesenchyme support that interacts with the other, tissue specific cell types. Thus, the growth and differentiation of cells within different tissues, and the development of tumors, are all dependent on mesenchymal functions.

### Knockout Mice

Loss-of-function experiments in mice are mostly done by the technique of gene knockout. Knock-out mice employed in the present invention demonstrate the important role played by immunoglobulin superfamily variants in hemopoiesis as exemplified herein below. The technology is well known in the art. It requires the use of mouse genes for the purpose of generating knockout of the specific gene in embryonic stem (ES) cells that are then incorporated into the mouse germ-line cells from which mice carrying the gene knockout are generated. From a human gene there are several ways to recover the homologous mouse gene. One way is to use the human gene to probe mouse genomic libraries of lambda phages, cosmids or BACs. Positive clones are examined and sequenced to verify the identity of the mouse gene. Another way is to mine the mouse EST database to find the matching mouse sequences. This can be the basis for generating primer-pairs or specific mouse probes that allow an efficient screen of the mouse genomic libraries mentioned above by PCR or by hybridization. For the vast majority of genes the mouse homologue of the human gene retains the same biological function. The loss-of-function experiments in mice indicate the consequences of absence of expression of the gene on the phenotype of the mouse and the information obtained is applicable to the function of the gene in humans. On many occasions a specific phenotype observed in knockout mice was similar to a specific human inherited disease and the gene then proved to be involved and mutated in the human disease.

### Antisense sequence

As will be exemplified herein below, the expression or lack of expression of the mesenchymal TCR and immunoglobulin heavy chains seems to control interactions of the mesenchyme with other neighboring cells, especially in the process of hemopoiesis. The invention therefore further relates to the use of the cDNA and antisense molecules of the invention derived from mesenchymal TCR mRNAs for expression in cells and tissues for the purpose of modulating stromal/mesenchymal interactions and cell-cell communication with their neighbors in the microenvironment of the tissue involved.

For this purpose, the cDNA or antisense molecule is inserted in appropriate vectors such as, but not limited to, the retroviral vectors DCA*l* and DCM*m* that have been used in clinical trials in gene therapy (Bordignon et al., 1995). Preferably, the vector containing the cDNA or the antisense molecule, under the control of a suitable promoter such as that cDNA's own promoter, will be used to infect or transfect suitable mammalian, preferably human, most preferably the patient's autologous mesenchymal cells. The genetically-modified mesenchymal cells are then administered to a patient in need thereof by an appropriate route and are expressed in the desired site or tissue.

In order to manipulate the expression of an undesirable gene, it is necessary to produce antisense RNA in a cell. To this end, the complete or partial cDNA of an undesirable gene in accordance with the present invention is inserted into an expression vector comprising a promoter. The 3' end of the cDNA is thereby inserted adjacent to the 3' end of the promoter, with the 5' end of the cDNA being separated from the 3' end of the promoter by said cDNA. Upon expression of the cDNA in a cell, an antisense RNA is therefore produced which is incapable of coding for the protein. The presence of antisense RNA in the cell reduces the expression of the cellular (genomic) copy of the undesirable gene.

For the production of antisense RNA, the complete cDNA may be used. Alternatively, a fragment thereof may be used, which is preferably between about 9 and 2,000 nucleotides in length, more preferably between 15 and 500 nucleotides, and most preferably between 30 and 150 nucleotides.

The fragment is preferably corresponding to a region within the 5' half of the cDNA, more preferably the 5' region comprising the 5' untranslated region and/or the first exon region, and most preferably comprising the ATG translation start site. Alternatively, the fragment may correspond to DNA sequence of the 5' untranslated region only.

A synthetic oligonucleotide may be used as antisense oligonucleotide. The oligonucleotide is preferably a DNA oligonucleotide. The length of the antisense oligonucleotide is preferably between 9 and 150, more preferably between 12 and 60, and most preferably between 15 and 50 nucleotides. Suitable antisense oligonucleotides that inhibit the production of the protein of the present invention from its encoding mRNA can be readily determined with only routine experimentation through the use of a series of overlapping oligonucleotides similar to a "gene walking" technique that is well-known in the art. Such a "walking" technique as well-known in the art of antisense development can be done with synthetic oligonucleotides to walk along the entire length of the sequence complementary to the mRNA in segments on the order of 9 to 150 nucleotides in length. This "gene walking" technique will identify the oligonucleotides that are complementary to accessible regions on the target mRNA and exert inhibitory antisense activity.

Alternatively, an oligonucleotide based on the coding sequence of a protein capable of binding to an undesirable gene or the protein encoded thereby can be designed using Oligo 4.0 (National Biosciences, Inc.). Antisense molecules may also be designed to inhibit translation of an mRNA into a polypeptide by preparing an antisense which will bind in the region spanning approximately -10 to +10 nucleotides at the 5' end of the coding sequence.

Modifications of oligonucleotides that enhance desired properties are generally used when designing antisense oligonucleotides. For instance, phosphorothioate bonds are used instead of the phosphoester bonds that naturally occur in DNA, mainly because such phosphorothioate oligonucleotides are less prone to degradation by cellular enzymes. Preferably, a 2'-methoxyribonucleotide modification in 60% of the oligonucleotides is used. Such modified oligonucleotides are capable of eliciting an antisense effect comparable to the effect observed with phosphorothioate oligonucleotides.

Therefore, the preferred antisense oligonucleotide of the present invention has a mixed phosphodiester-phosphorothioate backbone. Preferably, 2'-methoxyribonucleotide modifications in about 30% to 80%, more preferably about 60%, of the oligonucleotide are used.

In the practice of the invention, antisense oligonucleotides or antisense RNA may be used. The length of the antisense RNA is preferably from about 9 to about 3,000 nucleotides, more preferably from about 20 to about 1,000 nucleotides, most preferably from about 50 to about 500 nucleotides.

In order to be effective, the antisense oligonucleotides of the present invention must travel across cell membranes. In general, antisense oligonucleotides have the ability to cross cell membranes, apparently by uptake via specific receptors. As the antisense oligonucleotides are single-stranded molecules, they are to a degree hydrophobic, which enhances passive diffusion through membranes. Modifications may be introduced to an antisense oligonucleotide to improve its ability to cross membranes. For instance, the oligonucleotide molecule may be linked to a group, which includes a partially unsaturated aliphatic hydrocarbon chain, and one or more polar or charged groups such as carboxylic acid groups, ester groups, and alcohol groups. Alternatively, oligonucleotides may be linked to peptide structures, which are preferably membranotropic peptides. Such modified oligonucleotides penetrate membranes more easily, which is critical for their function and may, therefore, significantly enhance their activity.

### Introduction of Proteins, Peptides, and DNA into Cells

The present invention provides proteins encoded by the truncated immunoglobulin superfamily variant genes, peptides derived therefrom and antisense DNA molecules based on the variant gene transcripts. A therapeutic or research-associated use of these tools necessitates their introduction into cells of a living organism or into cultured cells. For this purpose, it is desired to improve membrane permeability of peptides, proteins and antisense molecules. The same principle, namely, derivatization with lipophilic structures, may also be used in creating peptides and proteins with enhanced membrane permeability. For instance, the sequence of a known membranotropic peptide may be added to the sequence of the peptide or protein. Further, the peptide or protein may be derivatized by partly lipophilic structures such as the above-noted hydrocarbon chains, which are substituted with at least one polar or charged group. For example, lauroyl derivatives of peptides have been described in the art. Further modifications of peptides and proteins include the oxidation of methionine residues to thereby create sulfoxide groups and derivatives wherein the relatively hydrophobic peptide bond is replaced by its ketomethylene isoester (COCH₂) have been described. It is known to those of skill in the art of protein and peptide chemistry these and other modifications enhance membrane permeability.

Another way of enhancing membrane permeability is to make use of receptors, such as virus receptors, on cell surfaces in order to induce cellular uptake of the peptide or protein. This mechanism is used frequently by viruses, which bind specifically to certain cell surface molecules. Upon binding, the cell takes the virus up into its interior. The cell surface molecule is called a virus receptor. For instance, the integrin molecules CAR and AdV have been described as virus receptors for Adenovirus. The CD4, GPR1, GPR15, and STRL33 molecules have been identified as receptors/ coreceptors for HIV.

By conjugating peptides, proteins or oligonucleotides to molecules that are known to bind to cell surface receptors, the membrane permeability of said peptides, proteins or oligonucleotides will be enhanced. Examples of suitable groups for forming conjugates are sugars, vitamins, hormones, cytokines, transferrin, asialoglycoprotein, and the like molecules. Low et al U.S. Patent 5,108,921 describes the use of these molecules for the purpose of enhancing membrane permeability of peptides, proteins and oligonucleotides, and the preparation of said conjugates.

Low and coworkers further teach that molecules such as folate or biotin may be used to target the conjugate to a multitude of cells in an organism, because of the abundant and nonspecific expression of the receptors for these molecules.

The above use of cell surface proteins for enhancing membrane permeability of a peptide, protein or oligonucleotide of the invention may also be used in targeting the peptide, protein or oligonucleotide of the present invention to certain cell types or tissues. For instance, if it is desired to target neural cells, it is preferable to use a cell surface protein that is expressed more abundantly on the surface of those cells.

The protein, peptide or oligonucleotide of the invention may therefore, using the above-described conjugation techniques, be targeted to mesenchymal cells. For instance, if it is desired to enhance mesenchymal cell growth in order to augment autologous or allogeneic bone marrow transplantation or wound healing, then the immunoglobulin superfamily variant genes could be inserted into mesenchymal cells as a form of gene therapy. In this embodiment, local application of the cells containing the cDNA molecule can be used to modulate mesenchymal cell-cell interactions with neighboring cells in the microenvironment thus enhancing the wound healing process

In contrast, it is often desirable to inhibit mesenchymal cell-cell interactions, as in the case of a tumor. Therefore, mesenchymal cells of the tumor can be transfected with the antisense cDNA and then be used for treatment of localized solid tumors, to achieve regression of the tumor by blocking mesenchyme intercellular communication.

The proteins encoded by the mRNAs of the invention are cell surface receptors of mesenchymal cells and may probably interact with ligands presented by neighboring hemopoietic or non-hemopoietic cells. Thus, in bound or soluble form, these proteins or the peptides derived therefrom, may have modulatory effects on cells that bear said ligands.

### Antibodies

The present invention also comprehends antibodies specific for the polypeptides or peptides encoded by the truncated immunoglobulin superfamily variant transcripts, which are part of the present invention as discussed above. The proteins and peptides of the invention may be used as immunogens for production of antibodies that may be used as markers of mesenchymal cells. Such an antibody may be used for diagnostic purposes to identify the presence of any such naturally-occurring proteins. Such antibody may be a polyclonal antibody or a monoclonal antibody or any other molecule that incorporates the antigen-binding portion of a monoclonal antibody specific for such a protein. Such other molecules may be a single-chain antibody, a humanized antibody, an F(ab) or F(ab')₂ fragment, a chimeric antibody, an antibody to which is attached a label, such as fluorescent or radioactive label, or an immunotoxin in which a toxic molecule is bound to the antigen binding portion of the antibody. The examples are intended to be non-limiting. However, as long as such a molecule includes the antigen-binding portion of the antibody, it will be expected to bind to the protein and, thus, can be used for the same diagnostic purposes for which a monoclonal antibody can be used.

### Pharmaceutical compositions

These compositions are for use by injection, topical administration, or oral uptake. Preferred uses of the pharmaceutical compositions of the invention topically (including intraocularly, vaginally, rectally, intranasally and by inhalation), orally, or parenterally, for example by intravenous drip or intraperitoneal, subcutaneous, or intramuscular injection.

The pharmaceutical composition of the invention generally comprises a buffering agent, an agent which adjusts the osmolarity thereof, and optionally, one or more carriers, excipients and/or additives as known in the art, e.g., for the purposes of adding flavors, colors, lubrication, or the like to the pharmaceutical composition.

Carriers are well known in the art and may include starch and derivatives thereof, cellulose and derivatives thereof, e.g., microcrystalline cellulose, xanthan gum, and the like. Lubricants may include hydrogenated castor oil and the like.

A preferred buffering agent is phosphate-buffered saline solution (PBS), which solution is also adjusted for osmolarity.

A preferred pharmaceutical formulation is one lacking a carrier. Such formulations are preferably used for administration by injection, including intravenous injection.

The preparation of pharmaceutical compositions is well known in the art and has been described in many articles and textbooks.

Additives may also be selected to enhance uptake of the antisense oligonucleotide across cell membranes. Such agents are generally agents that will enhance cellular uptake of double-stranded DNA molecules. For instance, certain lipid molecules have been developed for this purpose, including the transfection reagents DOTAP (Boehringer Mannheim), Lipofectin, Lipofectam, and Transfectam, which are available commercially. The antisense oligonucleotide of the invention may also be enclosed within liposomes.

The preparation and use of liposomes, e.g., using the above-mentioned transfection reagents, is well known in the art. Other methods of obtaining liposomes include the use of Sendai virus or of other viruses.

The above-mentioned cationic or nonionic lipid agents not only serve to enhance uptake of oligonucleotides into cells, but also improve the stability of oligonucleotides that have been taken up by the cell.

### Detection of Protein Products of Genes of Interest

Techniques for detecting a protein translation product of interest include, but are not limited to, immunoblotting or Western blotting, ELISA, sandwich assays, fluorescence, or biotin or enzymatic labeling with or without secondary antibodies.

Western blot analysis can be done on the tissue biopsies or tissue aspirates. This would involve resolving the proteins on an electrophoretic gel, such as an SDS PAGE gel, and transferring the resolved proteins onto a nitrocellulose or other suitable membrane. The proteins are incubated with a target binding molecule, such as an antibody.

This binding reagent may be labeled or not. If it were unlabeled, then one would also employ a secondary, labeled molecule which binds to the binding reagent. One approach involves avidinating one molecule and biotinylating the other. Another is for the secondary molecule to be a secondary antibody, which binds the original binding reagent.

To improve detection of the specific protein, immunoprecipitation can be conducted. This typically will involve addition of a monoclonal antibody against the protein of interest to samples, then allowing the Ig-protein complex to precipitate after the addition of an affinity bead (ie antihuman Ig Sepharose bead). The immunoprecipitates will undergo several washings prior to transfer onto a nitrocellulose membrane. The Western blot analysis can be performed using another antibody against the primary antibody used.

Having now generally described the invention, the same will be more readily understood through reference to the following example, which is provided by way of illustration and is not intended to be limiting of the present invention.

### EXAMPLES

### Primary cell cultures

(i) Bone marrow: Mouse bone marrow cells were obtained from femur and tibia of 1-2 week old female C57BL/6 mice. Bone marrow cells were removed aseptically by flushing culture medium through the marrow cavity using a 1ml syringe fitted with a 27-gauge needle. 1 x 10⁷ cells/ml were seeded in DMEM with 20% FCS (Bio Lab, Israel) and cultured for 4-5 days at 37°C and 5% CO₂ atmosphere. The plates were washed and covered with fresh culture medium. After 3 weeks, a monolayer was formed. The cells were passaged monthly at a split ratio of 1:10 using 0.5% trypsin (Sigma, St. Louis, MO) containing 0.02% EDTA.
(ii) Embryonic fibroblasts: Mouse embryo were minced in PBS solution and treated with 0.5% trypsin and 0.02% EDTA at 37°C for 15 minutes. The supernatant was collected and treated again with trypsin for 30 minutes. The cell suspension obtained was then washed a few times, resuspended in DMEM containing 10% FCS to a final concentration of 1 x 10⁶ cells/ml, and cultured for 4-5 days at 37°C and 5% CO₂ atmosphere. When a fibroblast monolayer was formed, it was trypsinized for 5 minutes, and the cells were washed and resuspended as indicated before. This cell suspension (2x10⁵ cells/ml) was cultured again for 4-5 days and then collected.
(iii) Thymus and liver cells were obtained from Balb/c mice, 6-10 weeks old.

### Long term hemopoietic cultures

Bone marrow cells from Balb/C mice at 5x10⁵ per culture, or 10³ FACS sorted CD34⁴ Lin⁻ cells, were seeded onto a confluent layer of MEF that were passaged prior to use 3-13 times *in vitro.* Bone marrow cells were seeded in α-MEM (Gibco-BRL, Paisley, UK) supplemented with 20% horse serum (StemCell Technologies, Vancouver, Canada) and 10⁻⁶ hydrocortisone hemisuccinate and were maintained at 33°C in a humidified atmosphere of 10% CO₂ in air. In each experiment, 6 individual cultures from each group were seeded. The cultures were demidepopulated and refed twice weekly. The cells collected were counted and seeded in semisolid methylcellulose medium supplemented with interleukin (IL)-3 (10 ng/ml), erythropoietin (3 units/ml), ckit ligand (50 ng/ml) and IL-6 (10 ng/ml). Ckit ligand was purchased from PeproTech Inc. Rocky Hill, NJ and the other cytokines from R&D Systems, Minneapolis, MN. Colonies were counted at day 8 in culture.

### Statistics:

Data are presented as the mean ± standard error of the mean. Student's t-test was performed to determine significance.

### Defective Hemopoiesis of MEF from TCRβ Deficient Mice

One commonly studied function of the mesenchyme is the capacity to form an *in vitro* microenvironment suitable for the self-renewal and differentiation of hemopoietic stem cells. We utilized this phenomenon to compare the ability of normal MEF to those from TCRβ^{-/-} mice in their ability to support long term *in vitro* hemopoiesis. Bone marrow cells from donor Balb/C mice (Figure 1A) or FACS sorted purified population of CD34⁺ Lin⁻ cells enriched for hemopoietic stem cells (Figure 1B-I&C) seeded onto confluent cultures of MEF from normal mice formed cobblestone areas within a few days culture and proliferated throughout one month of incubation within the MEF layer and in the liquid phase of the cultures. Hemopoietic colony formation assays detected in these cultures high incidence of hemopoietic progenitors (Figure 1D). These findings were identical for MEF passaged 3-13 times in culture. By sharp contrast, MEF from TCRβ^{-/-} mice at early passages had inferior capacity to support hemopoiesis (not shown) and entirely lost this capacity when passaged over 7 times (Figure 1A, B-II and C). It is noteworthy, that MEF from TCRβ^{-/-} and wild type animals proliferated at this stage with the same population doubling times and did not show signs of morphological transformation. It remains to be determined whether TCR directly interacts with hemopoietic stem cells or whether its effect on mesenchymal hemopoietic functions is mediated indirectly. Our findings indicate that TCRβ knockout does not only result in a T cell defect but also causes a primary mesenchymal malfunctioning.

### Mesenchymal Cells Express Other Members of the Immunoglobulin Superfamily

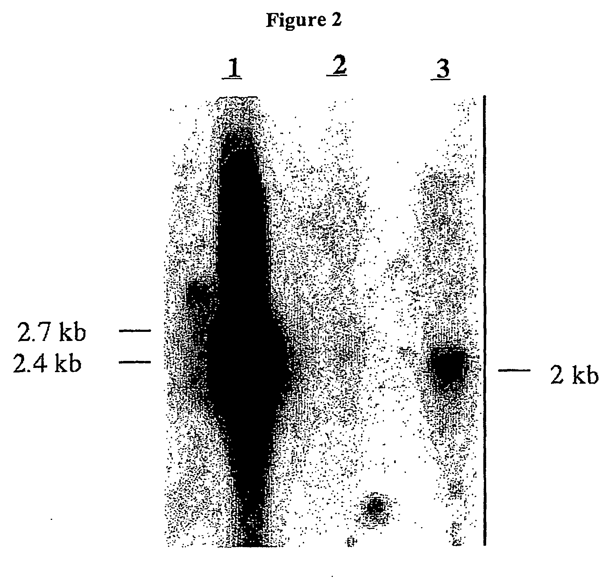

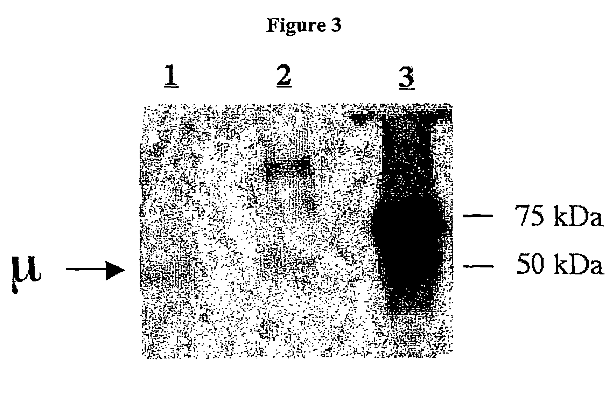

The finding that mesenchymal cells express TCR genes raised the possibility that other members of the immunoglobulin (Ig) superfamily are expressed in the mesenchyme. We screened a series of stromal mesenchymal cell lines derived in our laboratory including one subtype that shares properties with endothelial cells (MBA-2.1 cells). Using RT-PCR we discovered that the MBA-2.1 cells express an abundant amount of mouse µ chain mRNA. Northern blot analysis (Figure 2) verifies that this is the case. More importantly, we obtained evidence that the µ chain protein is expressed by this cell line. Indeed, Western blotting detected a band that corresponds to about 50 kDa in size (Figure 3) which is the size predicted by the mRNA sequence.

The truncated µ heavy chain (mouse)-MBA2.1 comprises the DNA sequence designated I+j2 [SEQ ID NO:1] which encodes peptide with the sequence [SEQ ID NO:2]. In addition, one form of the truncated µ heavy chain 5' begins from J4 and extends through the C region and contains either a transmembrane domain, [SEQ ID NO:3] or a cytoplasmic domain [SEQ ID NO:4]. Another form of the truncated µ heavy chain 5' begins from the constant region and contains either a transmembrane domain [SEQ ID NO:5] or a cytoplasmic domain [SEQ ID NO:6].

The case of the truncated µ heavy chain is very similar to the case of the truncated TCRβ. In the mesenchyme and endothelium, there is expression of Ig superfamily molecules that are truncated i.e. lacking the variable part and possessing a J region preceded by an intronic sequence including a codon for methionine. PCR analysis verified that MBA-2.1 cells do express such an mRNA. We thus find that at least one type, and possibly more, of stromal and endothelial cells express the Ig µ chain and may present this protein as a surface molecule. The expression of the µ chain by the mesenchyme and endothelium is expected to regulate or be associated with intercellular growth/differentiation control their neighboring cells. Both TCR and µ chain, are known to be linked to the cell growth/differentiation machinery and thus can be used to modulate menenchymal and endothelial intercellular interactions. The TCR appears to be most abundant in mesenchymal stroma whereas the µ chain seems to be abundant in endothelial stroma.

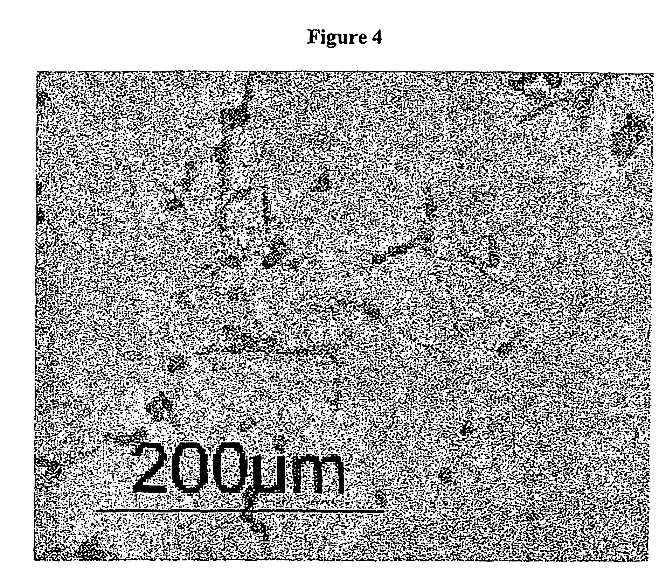

Lastly, to strengthen the results of the Western blot, Fig. 4 depicts immunostaining of MBA 2.1 cells with goat anti mouse- µ chain and amplified with biotinylated donkey anti goat and ABC kit

### Use of Mesenchymal and Endothelial TCR or Ig in Control of Neighboring Cell Growth or Differentiation

The cDNAs encoding these molecules can be used to control cell growth by either expressing the sense or the antisense sequences, depending on the intended purpose of the treatment. In the case of tumors the stroma enhances tumor growth. It is therefore desirable to inhibit or ultimately to block this effect of stromal cells on the tumor cells and thus it is appropriate to apply the antisense to either of the genes in order to shut off their expression. The same thing is true for diseases such as myelofibrosis when the bone marrow becomes loaded with mesenchymal cells that block normal hemopoiesis and should be eliminated.

In order to manipulate the expression of an undesirable gene, it is desirable to produce antisense RNA in a cell. To this end, the complete or partial cDNA of an undesirable gene in accordance with the present invention is inserted into an expression vector comprising a promoter. The 3' end of the cDNA is thereby inserted adjacent to the 3' end of the promoter, with the 5' end of the cDNA being separated from the 3' end of the promoter by said cDNA. Upon expression of the cDNA in a cell, an antisense RNA is therefore produced which is incapable of coding for the protein. The presence of antisense RNA in the cell reduces the expression of the cellular (genomic) copy of the undesirable gene.

For the production of antisense RNA, the complete cDNA may be used. Alternatively, a fragment thereof may be used, which is preferably between about 9 and 2,000 nucleotides in length, more preferably between 15 and 500 nucleotides, and most preferably between 30 and 150 nucleotides.

The fragment is preferably corresponding to a region within the 5' half of the cDNA, more preferably the 5' region comprising the 5' untranslated region and/or the first exon region, and most preferably comprising the ATG translation start site. Alternatively, the fragment may correspond to DNA sequence of the 5' untranslated region only.

A synthetic oligonucleotide may be used as antisense oligonucleotide. The oligonucleotide is preferably a DNA oligonucleotide. The length of the antisense oligonucleotide is preferably between 9 and 150, more preferably between 12 and 60, and most preferably between 15 and 50 nucleotides. Suitable antisense oligonucleotides that inhibit the production of the protein of the present invention from its encoding mRNA can be readily determined with only routine experimentation through the use of a series of overlapping oligonucleotides similar to a "gene walking" technique that is well-known in the art. Such a "walking" technique as well known in the art of antisense development can be done with synthetic oligonucleotides to walk along the entire length of the sequence complementary to the mRNA in segments on the order of 9 to 150 nucleotides in length. This "gene walking" technique will identify the oligonucleotides that are complementary to accessible regions on the target mRNA and exert inhibitory antisense activity.

Alternatively, an oligonucleotide based on the coding sequence of a protein capable of binding to an undesirable gene or the protein encoded thereby can be designed using Oligo 4.0 (National Biosciences, Inc.). Antisense molecules may also be designed to inhibit translation of an mRNA into a polypeptide by preparing an antisense which will bind in the region spanning approximately -10 to +10 nucleotides at the 5' end of the coding sequence.

Modifications of oligonucleotides that enhance desired properties are generally used when designing antisense oligonucleotides. For instance, phosphorothioate bonds are used instead of the phosphoester bonds that naturally occur in DNA, mainly because such phosphorothioate oligonucleotides are less prone to degradation by cellular enzymes Preferably, 2'-methoxyribonucleotide modifications in 60% of the oligonucleotide is used. Such modified oligonucleotides are capable of eliciting an antisense effect comparable to the effect observed with phosphorothioate oligonucleotides.

Therefore, the preferred antisense oligonucleotide of the present invention has a mixed phosphodiester-phosphorothioate backbone. Preferably, 2'-methoxyribonucleotide modifications in about 30% to 80%, more preferably about 60%, of the oligonucleotide are used.

In the practice of the invention, antisense oligonucleotides or antisense RNA may be used. The length of the antisense RNA is preferably from about 9 to about 3,000 nucleotides, more preferably from about 20 to about 1,000 nucleotides, most preferably from about 50 to about 500 nucleotides.

In order to be effective, the antisense oligonucleotides of the present invention must travel across cell membranes. In general, antisense oligonucleotides have the ability to cross cell membranes, apparently by uptake via specific receptors. As the antisense oligonucleotides are single-stranded molecules, they are to a degree hydrophobic, which enhances passive diffusion through membranes. Modifications may be introduced to an antisense oligonucleotide to improve its ability to cross membranes. For instance, the oligonucleotide molecule may be linked to a group, which includes a partially unsaturated aliphatic hydrocarbon chain, and one or more polar or charged groups such as carboxylic acid groups, ester groups, and alcohol groups. Alternatively, oligonucleotides may be linked to peptide structures, which are preferably membranotropic peptides. Such modified oligonucleotides penetrate membranes more easily, which is critical for their function and may, therefore, significantly enhance their activity.

The antisense oligonucleotides of the invention are generally provided in the form of pharmaceutical compositions. These compositions are for use by injection, topical administration, or oral uptake.

### Pharmaceutical Compositions

Preferred uses of the pharmaceutical compositions of the invention by injection are subcutaneous injection, intravenous injection, and intramuscular injection. Less convenient routes of administration may include intraperitoneal, intradural, intra-thecal administration or intra-arterial administration when required.

The pharmaceutical composition of the invention generally comprises a buffering agent, an agent which adjusts the osmolarity thereof, and optionally, one or more carriers, excipients and/or additives as known in the art, e.g., for the purposes of adding flavors, colors, lubrication, or the like to the pharmaceutical composition.

Carriers may include starch and derivatives thereof, cellulose and derivatives thereof, e.g., microcrystalline cellulose, xanthan gum, and the like. Lubricants may include hydrogenated castor oil and the like.

A preferred buffering agent is phosphate-buffered saline solution (PBS), which solution is also adjusted for osmolarity.

A preferred pharmaceutical formulation is one lacking a carrier. Such formulations are preferably used for administration by injection, including intravenous injection. The preparation of pharmaceutical compositions is well known in the art.

Additives may also be selected to enhance uptake of the antisense oligonucleotide across cell membranes. Such agents are generally agents that will enhance cellular uptake of double-stranded DNA molecules. For instance, certain lipid molecules have been developed for this purpose, including the transfection reagents DOTAP (Boehringer Mannheim), Lipofectin, Lipofectam, and Transfectam, which are available commercially. The antisense oligonucleotide of the invention may also be enclosed within liposomes. The preparation and use of liposomes, e.g., using the above-mentioned transfection reagents, is well known in the art.

### Gene therapy

On the other hand it may be important to increase the expression of the TCR gene in conditions requiring more augmented intercellular mesenchymal interactions such as in improper wound healing, for example by means of gene therapy. By the same token, the µ chain may have overlapping or complementary functions to TCR and may be used in a similar manner, for other purposes relating to endothelium, i.e. to suppress neo-vascularization in tumors on the one hand and to enhance vascularization in diseases that involve defective blood vessel formation, on the other. Our present experiments show that the TCR affects hemopoiesis, and it is likely that the µ chain has similar or complementary functions.

Recently, gene transfer into hematopoietic cells using viral vectors has focused mostly on lymphocytes and hematopoietic stem cells (HSCs). HSCs have been considered particularly important as target cells because of their pluripotency and ability to reconstitute hemopoiesis after myeloablation and transplantation. HSCs are believed to have the ability to live a long time, perhaps a lifetime, in the recipient following bone marrow transplantation. Genetic correction of HSCs can therefore potentially last a lifetime and permanently cure hematologic disorders in which genetic deficiencies cause the pathology. Oncoretroviral vectors have been the main vectors used for HSCs because of their ability to integrate into the chromosomes of their target cells. Gene-transfer efficiency of murine HSCs is high using oncoretroviral vectors. In contrast, gene-transfer efficiency using the same viral vectors to transduce human HSCs or HSCs from large animals has been much lower. Although these difficulties may have several causes, the main reason for the low efficiency of human HSC transduction with oncoretroviral vectors is probably because of the nondividing nature of HSCs. Murine HSCs can be easily stimulated to divide in culture, whereas it is more problematic to stimulate human HSCs to divide rapidly in vitro. Because oncoretroviral vectors require dividing target cells for successful nuclear import of the preintegration complex and subsequent integration of the provirus, only the dividing fraction of the target cells can be transduced.

In addition, adenovirus (Adv)-mediated gene transfer has recently gained new attention as a means to deliver genes for hematopoietic stem cell (HSC) or progenitor cell gene therapy. In the past, HSCs have been regarded as poor Adv targets, mainly because they lack the specific Adv receptors required for efficient and productive Adv infection. In addition, the nonintegrating nature of Adv has prevented its application to HSC and bone marrow transduction protocols where long-term expression is required. There is even controversy as to whether Adv can infect hematopoietic cells at all. In fact, the ability of Adv to infect epithelium-based targets and its inability to effectively transfect HSCs have been used in the development of eradication schemes that use Adv to preferentially infect and "purge" tumor cell-contaminating HSC grafts. However, there are data supporting the existence of productive Adv infections into HSCs. Such protocols involve the application of cytokine mixtures, high multiplicities of infection, long incubation periods, and more recently, immunological and genetic modifications to Adv itself to enable it to efficiently transfer genes into HSCs. This is a rapidly growing field, both in terms of techniques and applications.

Having now fully described certain preferred embodiments of this invention, it will be appreciated by those skilled in the art that the same can be performed within a wide range of equivalent parameters, concentrations, and conditions without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

Reference to known method steps, conventional method steps, known methods or conventional methods is not in any way an admission that any aspect, description or embodiment of the present invention is disclosed, taught or suggested in the relevant art

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### REFERENCES

Abbas et al (Eds.) (1994). Cellular and Molecular Immunology Chapter II, W.B. Saunders Co. (Philadelphia, USA).
Bagdy, G.C., Heinrich, M.C. (1991) Vascular endothelial cells and hematopoiesis regulation of gene expression in human vascular endothelial cells. *Hematol Pathol* 5,93-99
Barda-Saad, M, Shav-Tal, Y, Rozenszajn, AL, Cohen, M, Zauberman, A, Karmazyn, A, Parameswaran, R, Schori, H, Ashush, H, Ben-Nun, A, and Zipori, D (2002). The mesenchyme expresses T cell receptor mRNAs: relevance to cell growth control. Oncogene 21 : 2029-2036
Bordignon C, Notarangelo LD, Nobili N, Ferrari G, Casorati G, Panina P, Mazzolari E, Maggioni D, Rossi C, Servida P, et al. (1995). Gene therapy in peripheral blood lymphocytes and bone marrow for ADA- immunodeficient patients. Science 270(5235):470-5
Bornemann, K.D., Brewer, J.W., Perez, E., Doerre, S., Sita, R., Corley, R.B. (1997) Secretion of soluble pre-B cell receptors by pre-B cells. *J Immunol* **15**,2551-7
Calman AF, Peterlin BM (1986) Expression of T cell receptor genes in human B cells. J Exp Med 164(6):1940-57
Cheng, A.M., Rowley, B. Pao, W. Hayday, A. Bolen, J.B. Pawson, T. (1995) Syk tyrosine kinase required for mouse viability and B-cell development. *Nature* **378**,303
Ehlich, A., Martin, V., Müller, W. and Rajewsky, K. (1994) Analysis of the B cell progenitor compartment at the level of single cells. *Curr Biol* **4**, 573-583
Essand M, Vasmatzis G, Brinkmann U, Duray P, Lee B, Pastan I (1999).High expression of a specific T-cell receptor gamma transcript in epithelial cells of the prostate. Proc Natl Acad Sci USA 96(16):9287-92
Fagioli M, Care A, Ciccone E, Moretta L, Moretta A, Meccia E, Testa U, Falini B, Grignani F, Peschle C, et al. (1991). Molecular heterogeneity of the 1.0-kb T beta transcript in natural killer and gamma/delta lymphocytes. Eur. J Immunol. 21(6):1529-34
Fina, L., Molgaard, H.V., Robertson, D., Bradley, N.J., Monaghan, P., Delia, D., Sutherland, D.R., Baker, M.A.,and Greares, M.F. (1994) Expression of the CD34 gene in vascular endothelial cells. *Blood* 75, 2417-2426
Fleischman, R.A., Simpson, F., Gillardo, T., Jin, X., Perkins, S. (1995) Isolation of endothelial-like stromal cells that express kit ligand and support in vitro hematopoiesis. *Exp Hematol* **23***,* 1407-1416
Garcia Porrero, J.A., Godin, I.E., Dieterlen Lievre, F. (1995) Potential intraembryonic hemogenic sites at pre-liver stages in the mouse Anatomy and Embryology. Berlin **192**, 425-435.
Gong, S., and Nussenzweig, M.C. (1996) Regulation of an early developmental checkpoint in the B cell pathway by Igβ. *Science* **272,** 411-414
Hardy, R.R., Carmack, C.E., Shinton, S.A., Kemp, J.D. and Hayakawa, K. (1991) Resolution and characterization of pro-B and pre-pro B cell stages in normal mouse bone marrow. *J Exp Med* **173**, 1213-1225
Horne, M.C., Roth, P.E., DeFranco, A.L. (1996) Assembly of the truncated immunoglobulin heavy chain Dµ into antigen receptor-like complexes in pre-B cells but not in B cells. *Immunity* **4**,145-58
Ichihara, Y., Hayashida, H., Miyazawa, S., and Kurosawa, Y. (1989) Only DFL16, DSP2, and DQ52 gene families exist in mouse immunoglobulin heavy chain diversity gene loci, of which DFL16 and DSP2 originate from the same primordial DH gene. *Eur J Immunol* **19**, 1849-1854
Jameson SC, Bevan MJ (1995). T cell receptor antagonists and partial agonists. Immunity 2(1):1-11
Kincade, P. (1981) Formation of B lymphocytes in fetal and adult life. *Adv Immunol* 31,177
Kitamura, D., Roes, J., Kuhn, R., and Rajewsky, K.A. (1991) B cell-deficient mouse by targeted disruption of the membrane exon of the immunoglobulin µ chain gene. *Nature* 350,423-6
Kitamura, D., Kudo, A., Schaal, S., Muller, W., Melchers, F., and Rajewsky, K. (1992a) A critical role of lambda 5 protein in B cell development. *Cell* 69,823-31
Kitamura, D., Rajewsky, K., (1992b) Targeted disruption of µ chain membrane exon causes loss of heavy-chain allelic exclusion. *Nature* **356**,154-6
Lin, G., Finger, E., and Gutierrez-Ramos, J. (1995) Expression of CD34 in endothelial cells, hematopoietic progenitors and nervous cells in fetal and adult mouse tissues. *Eur J Immunol* **25***,* 1508-1515
Loffert, D., Ehlich, A., Muller, W., and Rajewsky, K. (1996) Surrogate light chain expression is required to establish immunoglobulin heavy chain allelic exclusion during early B cell development. *Immunity* **4** 133-44
Madrenas J, Pazderka F, Baergen C, Halloran PF (1991). Isolation of a murine renal cell population which expresses a truncated T-cell receptor-alpha mRNA. Transplant Proc 23(1 Pt 1):837-8
Madrenas J, Pazderka F, Parfrey NA, Halloran PF (1992). Thymus-independent expression of a truncated T cell receptor-alpha mRNA in murine kidney. J Immunol. 148(2):612-9
Madrenas J, Vincent DH, Kriangkum J, Elliott JF, Halloran PF (1994). Alternatively spliced, germline J alpha 11-2-C alpha mRNAs are the predominant T cell receptor alpha transcripts in mouse kidney. Mol Immunol. 31(13):993-1004
Melchers, F., Karasuyama, H., Haasner, D., Bauer, S., Kudo, A., Sakaguchi, N., Jameson, B. and Rolink, A. (1993) The surrogate light chain in B cell development. *Immunol Today* **14**, 60-68
   Melchers, F. and Rolink, A. (1999) B-Lymphocyte Development and Biology. In Paul, W. E. (Ed.), Fundamental Immunology. 4^{th} Ed. *Lippincott-Raven Philadelphia 183-224*
Mombaerts, P., Iacomini, J., Johnson, R., Herrup, K., Tonegawa, S., and Papaioannou, V. (1992) RAG-1-deficient mice have no mature B and T lymphocytes. *Cell* **68**, 869-877
Osmond, D.G. (1990) B cell development in the bone marrow. *Semin Immunol* **2**, 173-180
Palacios, R., and Samaridis, J. (1992) Fetal liver pro-B and pre-B lymphocyte clones: expression of lymphoid-specific genes, surface markers, growth requirements, colonization of the bone marrow, and generation of B lymphocytes in vivo and in vitro. *Mol Cell Biol* **12**,518-30
Qian L, Vu MN, Carter MS, Doskow J, Wilkinson MF (1993). T cell receptor-beta mRNA splicing during thymic maturation in vivo and in an inducible T cell clone in vitro. J Immunol 15;151(12):6801-14
Rafii, S., Shapiro, F., Rimarachin, J., Nachman, R.L., Ferris, B., Weksler, B., Moore, M.A.S., and Asch, A.S. (1994) Isolation and characterization of human bone marrow microvascular endothelial cells: hematopoietic progenitor cells adhesion. *Blood* **84**, 10-19
Reth, M.G., and Alt, F.W. (1984) Novel immunoglobulin heavy chains are produced from DJH gene segment rearrangements in lymphoid cells. Nature **312**,418-23
Rogers, J., Early, P., Carter, C., Calame, K., Bond, M., Hood, L., and Wall, R. (1980) Two mRNAs with different 3' ends encode membrane-bound and secreted forms of immunoglobulin mu chain. *Cell* **20**,303-12
Rolink, A.G., Winkler, T., Melchers, F. and Andersson, J.(2000) Precursor B cell receptor-dependent B cell proliferation and differentiation does not require the bone marrow or fetal liver environment. *J Exp Med* **191,** 23-32
Shinkai, Y., Rathburn, G., Lam, K., Oltz, E.M., Stewart V., Mendelsohn, M., Charron, J., Datta, M., Young, F., Stall, A.M., and Alt, R. (1992) RAG-2-deficient mice lack mature lymphocytes owing to inability to initiate V(D)J rearrangment. *Cell* **68,** 855―867
Spanopoulou, E., C.A., Roman, L.M., Corcoran, M.S., Schlissel, D.P.1 Silver, D., Nemazee, M.C., Nussenzweig, S.A., Shinton, R.R., Hardy, and Baltimore. D. (1994) Functional immunoglobulin transgenes guide ordered B-cell differentiation in Rag-1-deficient mice. *Genes Dev* **8,** 1030-1042
Strominger JL (1989). Developmental biology of T cell receptors. Science **244**(4907):943-50
ten Boekel, E., Melchers, F., and Rolink, A.G. (1997)Changes in the V(H) gene repertoire of developing precursor B lymphocytes in mouse bone marrow mediated by the pre-B cell receptor. *Immunity* **7**, 357-368
Tornberg, U.C., Bergqvist, I., Haury, M" Holmberg, D. (1998) Regulation of B lymphocyte development by the truncated immunoglobulin heavy chain protein Dµ *J Exp Med* **187**,703-9
Torres, R.M., Flaswinkel, H., Reth, M., and Rajewsky. K. (1996) Aberrant B cell development and immune response in mice with a compromised BCR complex. *Science* **272**,1804-1808
Turner, M., Mee, P.J., Costello, P.S.,Williams, O., Price, A.A., Duddy, L.P., Furlong, M.T., Geahlen, R.L., and Tybulewicz, V.L.J. (1995) Perinatal lethality and blocked B-cell development in mice lacking the tyrosine kinase Syk. *Nature* **378**: 298-302
Wientroub S, Zipori D. (1996). "Stem Cell Culture" in: Principles of Bone Biology. J. Bilezikian, L. Raisz, G. Rodan, J. Markovac (eds). Academic Press, San Diego, pp 1267
Wolfgang CD, Essand M, Vincent JJ, Lee B, Pastan I (2000). TARP: a nuclear protein expressed in prostate and breast cancer cells derived from an alternate reading frame of the T cell receptor gamma chain locus. Proc Natl Acad Sci USA 97(17):9437-42
Yoshikai Y, Anatoniou D, Clark SP, Yanagi Y, Sangster R, Van den Elsen P, Terhorst C, Mak TW (1984). Sequence and expression of transcripts of the human T-cell receptor beta-chain genes. Nature **312**(5994):521-4
Young, F., Ardman, B., Shinkai, Y., Lansford, R, Blackwell, T.K., Mendelsohn, M., Rolink, A., Melchers, F., and Alt. F.W. (1994) Influence of immunoglobulin heavy- and light-chain expression on B-cell differentiation. *Genes Dev* **8,** 1043-1057
Zipori D (1989) Cultured stromal cell lines from hemopoietic tissues.In:Tavassoli M, ed, Blood Cell Formation: The Role of the Hemopoietic Microenvironment, Humana Press (Clifton, NY), p. 287
Zipori D (1990). Stromal cells in tumor growth and regression. Cancer J 3: 164
Zipori D, Tamir M (1989). Stromal cells of hemopoietic origin. Int J Cell Cloning 7(5):281-91

## Claims

1. An isolated polynucleotide comprising a transcript of an Immunoglobulin (Ig) gene, said polynucleotide lacking V region sequences and comprising a constant (C) domain and joining (J) region sequences, and a 5' intronic J sequence upstream to said J region sequence including an in-frame methionine codon, wherein said polynucleotide is selected from the group consisting of SEQ ID NO : 1 and SEQ ID NO : 3 to 6.

2. The polynucleotide according to claim 1, wherein the polynucleotide with the SEQ ID NO : I encodes a peptide consisting of the sequence SEQ ID NO : 2.

3. An antisense DNA molecule of any of the polynucleotides 1 to 2.

4. An expression vector comprising a polynucleotide or an antisense DNA molecule according to any one of the claims 1 to 3.

5. A host cell comprising a vector according to claim 4, wherein the host is a mammalian cell.

6. A host cell according to claim 5, which is a transfected mesenchymal human cell.

7. A polypeptide encoded by a polynucleotide according to any one of the claims 1 to 2.

8. Use of a vector according to claim 4 for transfection of mesenchymal cells.

9. Use of transfected mesenchymal human cells for the preparation of a medicament for inducing wound healing or for inducing hemopoieses after bone marrow transplantation or chemotherapy, wherein the transfected mesenchymal human cells comprising a polynucleotide comprising a transcript of a TCR or Immunoglobulin (Ig) gene, said polynucleotide lacking V region sequences and comprising a constant (C) domain and joining (J) region sequences, and a 5' intronic J sequence upstream to said J region sequence including an in-frame methionine codon, wherein the polynucleotide comprising a 5' intronic J sequence encodes a peptide selected from any one of SEQ ID NOs:7-42.

10. The use according to claim 9, wherein the cells are of an autologous origin.

11. The use according to claim 9, wherein the cells are of an allogeneic origin.

12. Use of transfected mesenchymal human cells for the preparation of a medicament for the suppression of cancer, wherein said transfected mesenchymal human cells comprising a DNA molecule according to claim 3, in an amount effective to suppress mesenchymal intercellular interactions.

13. The use according to claim 12, wherein the cells are of an autologous origin.

14. The use according to of claim 12, wherein the cells are of an allogeneic origin.

## Patentansprüche

1. Isoliertes Polynukleotid, welches ein Transkript eines Immunglobulin (Ig) Gens umfasst, wobei dem Polynukleotid die Sequenzen für die V-Region fehlen, und welches Sequenzen für eine konstante (C) Domäne und einen Verbindungs-(J) Bereich, und eine 5' intronische-J-Sequenz stromaufwärts der Sequenz des J-Bereichs einschließlich eines Methionin-Codons im Leseraster umfasst, wobei das Polynukleotid ausgewählt ist aus der Gruppe bestehend aus SEQ ID NO: 1 und SEQ ID NO: 3 bis 6.

2. Polynukleotid nach Anspruch 1, worin das Polynukleotid mit der SEQ ID NO: 1 ein Peptid codiert, das aus der Sequenz der SEQ ID NO: 2 besteht.

3. Antisense DNA-Molekül von einem der Polynukleotide 1 bis 2.

4. Expressions-Vektor, welcher ein Polynukleotid oder ein antisense DNA-Molekül nach einem der Ansprüche 1 bis 3 umfasst.

5. Wirts-Zelle, welche einen Vektor nach Anspruch 4 umfasst, wobei der Wirt eine Säugerzelle ist.

6. Wirts-Zelle nach Anspruch 5, welche eine transfizierte, mesenchymale menschliche Zelle ist.

7. Polypeptid, welches von einem Polynukleotid nach einem der Ansprüche 1 bis 2 codiert wird.

8. Verwendung eines Vektors nach Anspruch 4 zur Transfektion von mesenchymalen Zellen.

9. Verwendung von transfizierten, mesenchymalen menschlichen Zellen zur Herstellung eines Medikaments zur Induzierung von Wundheilung oder zur Induzierung von Hämopoiese nach Knochenmarkstransplantation oder Chemotherapie, wobei die transfizierten, mesenchymalen menschlichen Zellen ein Polynukleotid umfassen, welches ein Transkript eines TCR- oder Immunglobulin (Ig) Gens umfasst, worin dem Polynukleotid die Sequenzen der V-Region fehlen, und welches Sequenzen für eine konstante (C) Domäne und einen Verbindungs-(J) Bereich, und eine 5' intronische-J-Sequenz stromaufwärts der Sequenz des J-Bereichs einschließlich eines Methionin-Codons im Leseraster umfasst, worin das Polynukleotid eine 5' intronische-J-Sequenz enthält, welche ein Peptid codiert, ausgewählt aus einer der SEQ ID NOs: 7 bis 42.

10. Verwendung nach Anspruch 9, wobei die Zellen autologen Ursprungs sind.

11. Verwendung nach Anspruch 9, wobei die Zellen allogeneischen Ursprungs sind.

12. Verwendung von transfizierten, mesenchymalen menschlichen Zellen zur Herstellung eines Medikaments, zur Supprimierung von Krebs, wobei die transfizierten, mesenchymalen menschlichen Zellen ein DNA-Molekül nach Anspruch 3 in einer Menge umfassen, welche zur Supprimierung von mesenchymalen interzellulären Interaktionen wirksam ist.

13. Verwendung nach Anspruch 12, wobei die Zellen autologen Ursprungs sind.

14. Verwendung nach Anspruch 12, wobei die Zellen allogeneischen Ursprungs sind.

## Revendications

1. Un polynucléotide isolé comprenant une transcription d'un gène d'immunoglobuline (Ig), ledit polynucléotide étant dépourvu de séquences de région V et comprenant un domaine constant (C) et des séquences de région de jonction (J), et une séquence d'intron 5' J en amont de ladite séquence de région J incluant un codon méthionine encadré dans lequel ledit polynucléotide est choisi parmi le groupe consistant en SEQ ID NO : 1 et SEQ ID NO : 3 à 6.

2. Le polynucléotide selon la revendication 1, dans lequel le polynucléotide possédant la SEQ ID NO : 1 code un peptide consistant en la séquence SEQ ID NO : 2.

3. Une molécule ADN anti-sens de l'un quelconque des polynucléotides 1 à 2.

4. Un vecteur d'expression comprenant un polynucléotide ou une molécule ADN anti-sens selon l'une quelconque des revendications 1 à 3.

5. Une cellule hôte comprenant un vecteur selon la revendication 4, dans laquelle l'hôte est une cellule de mammifère.

6. Une cellule hôte selon la revendication 5, qui est une cellule mésenchymateuse humaine infectée.

7. Un polypeptide codé par un polynucléotide selon l'une quelconque des revendications 1 ou 2.

8. Utilisation d'un vecteur selon la revendication 4 pour l'infection de cellules

9. Utilisation de cellules mésenchymateuses humaines infectées pour la préparation d'un médicament pour induire la guérison de blessures ou pour induire l'hématopoïèse après transplantation de moelle osseuse ou chimiothérapie, dans laquelle les cellules mésenchymateuses humaines infectées comprennent un polynucléotide comprenant une transcription d'un TCR ou d'un gène d'immunoglobuline (Ig), ledit polynucléotide étant dépourvu de séquences de région V et comprenant un domaine constant (C) et des séquences de région de jonction (J), et une séquence d'intron 5' J en amont de ladite séquence de région J incluant un codon méthionine encadré, dans laquelle le polynucléotide comprenant une séquence d'intron 5' J code un peptide choisi parmi l'une quelconque des SEQ ID NOs : 7 - 42.

10. L'utilisation selon la revendication 9, dans laquelle les cellules sont d'une origine autologue.

11. L'utilisation selon la revendication 9, dans laquelle les cellules sont d'une origine allogène.

12. Utilisation de cellules mésenchymateuses humaines infectées pour la préparation d'un médicament pour la suppression du cancer, dans laquelle lesdites cellules mésenchymateuses humaines infectées comprennent une molécule ADN selon la revendication 3 en une quantité efficace pour supprimer des interactions intercellulaires mésenchymateuses.

13. L'utilisation selon la revendication 12, dans laquelle les cellules sont d'une origine autologue.

14. L'utilisation selon la revendication 12, dans laquelle les cellules sont d'une origine allogène.
